(19) Europäisches Patentamt
European Patent Office
Office européen des brevets

(11) EP 2 114 952 B1

(12) **EUROPÄISCHE PATENTSCHRIFT**

(45) Veröffentlichungstag und Bekanntmachung des Hinweises auf die Patenterteilung:
**10.08.2011 Patentblatt 2011/32**

(21) Anmeldenummer: **07858074.3**

(22) Anmeldetag: **21.12.2007**

(51) Int Cl.:
***C07D 493/04*** *(2006.01)* ***C08K 5/15*** *(2006.01)*

(86) Internationale Anmeldenummer:
**PCT/EP2007/064463**

(87) Internationale Veröffentlichungsnummer:
**WO 2008/095571 (14.08.2008 Gazette 2008/33)**

(54) **Gemisch von Diestern von Dianhydrohexitolderivaten mit Carbonsäuren der Summenformel C8H17COOH, Verfahren zur Herstellung dieser Diester und Verwendung dieser Gemische**

Mixture of diesters of dianhydrohexitol derivatives with carboxylic acids of the empirical formula C8H17COOH, processes for preparing these diesters and use of these mixtures

Mélanges de diesters de dérivés de dianhydrohexitol avec des acides carboxyliques de formule générale C8H17COOH, procédé de production de ces diesters, et utilisation de ces mélanges

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**RS**

(30) Priorität: **05.02.2007 DE 102007006442**

(43) Veröffentlichungstag der Anmeldung:
**11.11.2009 Patentblatt 2009/46**

(73) Patentinhaber: **Evonik Oxeno GmbH**
**45772 Marl (DE)**

(72) Erfinder:
- **GRASS, Michael**
  **45721 Haltern Am See (DE)**
- **SCHOLZ, Norbert**
  **45657 Recklinghausen (DE)**
- **KAIZIK, Alfred**
  **45772 Marl (DE)**
- **BÜSCHKEN, Wilfried**
  **45721 Haltern Am See (DE)**
- **LÜKEN, Hans-Gerd**
  **45770 Marl (DE)**

(56) Entgegenhaltungen:
**WO-A-99/45060**

- **HACHIHAMA Y ET AL: "Preparation of plasticizers from carbohydrate sources. I. Levulinic acid esters. II. Sorbide esters" TECHNOLOGY REPORTS OF THE OSAKA UNIVERSITY, OSAKA, JP, Bd. 3, Nr. 72, 1953, Seiten 191-200, XP008089789**
- **DATABASE CAPLUS [Online] CHEMICAL ABSTRACTS SERVICE, COLUMBUS, OHIO, US; XP002474595 gefunden im STN Database accession no. 1996:612518 & JP 08 188770 A (LION CORP) 23. Juli 1996 (1996-07-23)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 114 952 B1

## Beschreibung

Die vorliegende Erfindung betrifft ein Gemisch von Diestern von Dianhydrohexitolderivaten mit Carbonsäuren der Summenformel $C_8H_{17}COOH$, insbesondere von Isosorbidestern dieser Carbonsäuren. Ebenfalls betrifft die vorliegende Erfindung ein Verfahren zur Herstellung solcher Ester bzw. Gemische und deren Verwendung.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Düsononylphthalat (DINP) und Düsodecylphthalat (DIDP) Verwendung finden. Daneben werden beispielsweise auch alicyclische Polycarbonsäureester, wie die Ester der Cyclohexan-1,2-dicarbonsäure als Weichmacher für Kunststoffe wie Polyvinylchlorid (PVC), Polyvinylbutyral (PVB) und Polyolefine eingesetzt, da sie in Bezug auf die Gesundheitsgefährdung als unbedenklicher eingeschätzt werden, als die entsprechenden Phthalsäureester. Weiterhin können die vorgenannten Ester als Schmierölkomponente oder als Hilfsmittel in der Metallverarbeitung verwendet werden.

Die vorgenannten aromatischen oder aliphatischen Polycarbonsäureester basieren vollständig auf fossilen Rohstoffen, die nur eine begrenzte Verfügbarkeit aufweisen. Zur Schonung der fossilen Ressourcen besteht deshalb ein Bedarf an Polycarbonsäureestern, die zumindest teilweise auf nachwachsenden Rohstoffen basieren.

Hachihama, Y. et al stellen in "Preparation of plasticizers from carbohydrate sources. I. Levulinic acid esters. II. Sorbide esters" TECHNOLOGY REPORTS OF THE OSAKA UNIVERSITY, OSAKA, JP, Bd. 3, Nr. 72, 1953, S. 191 - 200, XP008089789 Diester aus Isosorbid und Carbonsäuren vor. Seite 198 offenbart in Tabelle 2 verschiedene Diester des Isosorbid mit Carbonsäuren. So zeigt die Nr. 10 z. B. den Ester des Isosorbid mit Decanoylsäure. Die Tabelle 4 gibt die Verwendung dieser Ester als Weichmacher in PVC an. Aus WO 99/45060 ist die Verwendung von Isosorbidderivaten, insbesondere auch von Isosorbidestern als Weichmacher z. B. für Polyvinylchlorid (PVC) bekannt. Zur Herstellung der Ester wird Isosorbid mit den entsprechenden Carbonsäuren umgesetzt. Die Carbonsäurereste können von 3 bis 12 Kohlenstoffatome aufweisen, wobei als mögliche Carbonsäurereste explizit Butanoyl, Hexanoyl, 2-Ethylhexanoyl, Octanoyl und Decanoyl genannt werden. In den Beispielen wird die Herstellung von Isosorbid-dioctanoat, Isosorbiddibutanoat, Isosorbid-dihexanoat und Isosorbid-bis(2-ethylhexanoat) beschrieben.

In WO 01/83488 wird ein Verfahren zur Herstellung von Isosorbidestern beschrieben, bei dem Dianhydroglycitol, Monoanhydroglycitol oder Glycitol mit der entsprechenden Carbonsäure, die vorzugsweise 3 bis 20 Kohlenstoffatome aufweist, in Gegenwart eines makroporösen, sauren Ionenaustauscherharzes umgesetzt wird. Dabei beträgt das molare Verhältnis von ((Di)anhydro)Glycitol zu Carbonsäure zwischen 2 und 5. In der Beschreibung wird ausgeführt, dass die Umsetzung mit verzweigten oder unverzweigten Säuren durchgeführt werden kann. Beispielhaft werden als mögliche Säuren Propansäure, Hexansäure, Octansäure, Nonansäure oder Decansäure genannt. In den Beispielen wird Octansäure oder 2-Ethylhexansäure mit Isosorbid umgesetzt.

Ausgehend von dem bekannten Stand der Technik bestand die Aufgabe der vorliegenden Erfindung in der Bereitstellung von alternativen Isosorbidestern, die als Weichmacher insbesondere zur Weichmachung von PVC gut geeignet sind.

Bei der Verwendung der beiden kommerziell verfügbaren Nonansäuren Pelargonsäure (n-Nonansäure) und 3,5,5-Trimethylhexansäure zur Herstellung der entsprechenden Dianhydrohexitolderivate wurde festgestellt, dass die so erhältlichen Ester nur bedingt als Weichmacher für PVC einsetzbar sind

Überraschenderweise wurde aber gefunden, dass Dianhydrohexitoldiester, insbesondere Isosorbiddiester, von Carbonsäuren mit 9 Kohlenwasserstoffen, die eine Mischung von zumindest zwei strukturell unterschiedlichen Nonansäuren enthalten und vorzugsweise einen bestimmten Verzweigungsgrad aufweisen, besonders gut als Weichmacher, insbesondere als Weichmacher für PVC geeignet sind.

Gegenstand der vorliegenden Erfindung ist deshalb ein Gemisch enthaltend Diester der Formel **I** eines Dianhydrohexitolderivates mit Carbonsäuren der Summenformel $C_8H_{17}COOH$

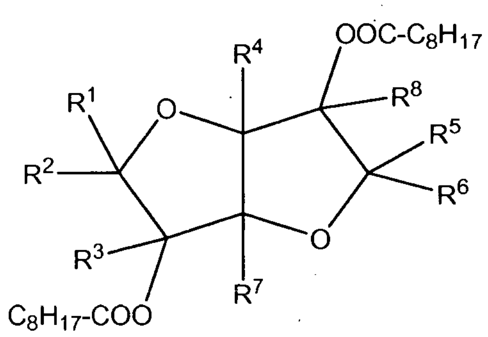

I

mit $R^1$ bis $R^8$ = H oder Alkylgruppe mit 1 bis 6 Kohlenstoffatomen, wobei die Reste $R^1$ bis $R^8$ gleich oder unterschiedlich sein können, welches dadurch gekennzeichnet ist, dass im Gemisch zumindest zwei unterschiedliche Diester **I** enthalten sind, die sich in der Konstitution zumindest eines der vorhandenen Carbonsäurereste $C_8H_{17}COO$ unterscheiden.

Ebenfalls Gegenstand der vorliegenden Erfindung ist ein Verfahren zur Herstellung von Diestern der Formel **I**, welches dadurch gekennzeichnet ist, dass ein sechswertiger Alkohol der Formel **II**,

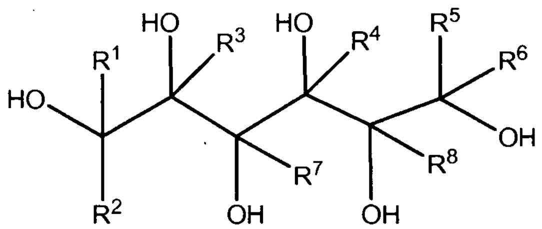

II

wobei die Reste $R^1$ bis $R^8$ die in Formel **I** genannte Bedeutung haben, und/oder ein Anhydro- oder Dianhydro-Derivat eines Alkohols der Formel **II** mit einem Gemisch, das zumindest zwei unterschiedliche Carbonsäuren der Summenformel $C_8H_{17}COOH$ aufweist, umgesetzt wird.

Außerdem ist Gegenstand der vorliegenden Erfindung die Verwendung der erfindungsgemäßen Gemische in Farben, Tinten oder Lacken, in Plastisolen, Klebstoffen oder Klebstoffkomponenten, in Dichtungsmassen, als Weichmacher in Kunststoffen oder Kunststoffkomponenten, als Lösemittel, als Schmierölkomponente und als Hilfsmittel bei der Metallverarbeitung sowie eine PVC-Zusammensetzung oder ein Plastisol, enthaltend PVC und von 5 bis 250 Massenteilen des erfindungsgemäßen Gemisches pro 100 Massenteile PVC. Die erfindungsgemäßen Gemische haben den Vorteil, dass sie zum Teil auf nachwachsenden Rohstoffen basieren und somit auch in Zukunft eine Verfügbarkeit gewährleistet ist. Ersten Untersuchungen zu Folge weisen Isosorbiddialkanoylester auch in der Regel vorteilhafte toxikologische Eigenschaften auf (van Haveren et al., ACS symposium series 2006, Vol 921, Seiten 99 bis 115). Weiterhin ist für den Fall, dass keine oder nur eine geringe Anzahl quartärer C-Atome im Säurerest vorliegt, eine gute biologische Abbaubarkeit zu erwarten.

Gegenüber Gemischen, enthaltend Verbindungen der Formel I, die auf nur einem Carbonsäure-Isomer basieren bzw. deren Carbonsäurereste einen Verzweigungsgrad von kleiner 0,7 aufweisen, zeichnen sich die erfindungsgemäßen Gemische in der Regel durch bessere Mischbarkeit mit PVC aus. Beispielsweise ist der Isosorbiddiester der n-Nonansäure (Pelargonsäure) bei Raumtemperatur fest, was eine Verwendung in Plastisolanwendungen erschwert bzw. ausschließt. Gegenüber Gemischen enthaltend Verbindungen der Formel I, die einen Verzweigungsgrad der Carbonsäurereste von größer 2,0 aufweisen, zeichnen sich die erfmdungsgemäßen Gemische durch deutlich verbesserte Tieftemperatureigenschaften (Flexibilisierung des Kunststoffes bei niedrigen Temperaturen) und niedrigere Viskosität in Plastisolen aus.

Wird im Rahmen der vorliegenden Erfindung von Isononansäure oder Isononanol gesprochen, werden darunter immer ein Isomer oder ein Gemisch von Isomeren der Nonansäure bzw. des Nonanols verstanden. Isononansäure bzw. Isononanol kann somit sowohl verzweigte als auch unverzweigte Isomere aufweisen.

Die erfindungsgemäßen Gemische enthaltend Diester eines Dianhydrohexitolderivates mit Carbonsäuren der Summenformel $C_8H_{17}COOH$ der Formel **I**

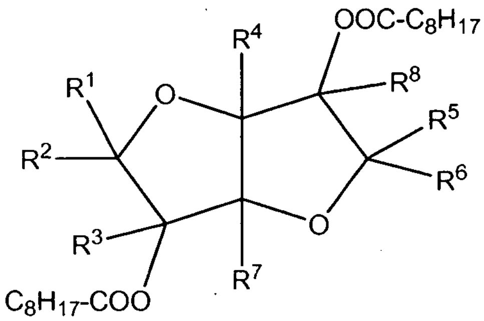

I

mit $R^1$ bis $R^8$ = H oder Alkylgruppe mit 1 bis 6 Kohlenstoffatomen, wobei die Reste $R^1$ bis $R^8$ gleich oder unterschiedlich sein können, zeichnen sich dadurch aus, dass im Gemisch zumindest zwei unterschiedliche Diester **I** enthalten sind, die sich in der Konstitution zumindest einer der vorhandenen und/oder beider vorhandenen Carbonsäurereste $C_8H_{17}COO$ unterscheiden. Die Gemische können dabei Diester aufweisen, bei denen einer oder beide Carbonsäurereste unverzweigte, einfach verzeigte und/oder mehrfach verzweigte Carbonsäurereste sind.

Vorzugsweise sind die erfindungsgemäßen Gemische so zusammengesetzt, dass die durch Verseifung der Diestergemische erhaltenen Carbonsäuren mindestens zwei Carbonsäuren der Summenformel $C_8H_{17}COOH$ mit unterschiedlicher Konstitutionsformel aufweisen, wobei keine der im Gemisch vorhandenen Carbonsäuren einen Anteil von mehr als 95 Mol-%, bevorzugt zumindest 90 Mol-%, aufweist.

Es kann vorteilhaft sein, wenn die durch Verseifung der im erfindungsgemäßen Gemisch enthaltenen Diester der Formel I erhaltenen Carbonsäuren der Summenformel $C_8H_{17}COOH$ weniger als 10 Mol-%, bevorzugt weniger 5 Mol-%, besonders bevorzugt weniger als 1 Mol-% und ganz besonders bevorzugt von 0,0001 bis 1 Mol-% an 3,5,5-Trimethylhexansäure oder anderen dreifach substituierten Nonansäuren, insbesondere solche mit quartären C-Atomen aufweisen.

Bevorzugt weisen Gemische von Carbonsäuren der Summenformel $C_8H_{17}COOH$, die durch Verseifung der erfindungsgemäßen Gemische von Diestern der Formel I erhalten werden, weniger als 1 Mol-%, vorzugsweise weniger als 0,001 Mol-% und bevorzugt keine Säuren auf, die ein quartäres Kohlenstoffatom aufweisen. Dies hat den Vorteil, dass die entsprechenden Säuren oder Ester leichter biologisch abgebaut werden können und somit eine bessere Umweltbilanz zeigen.

Es kann außerdem vorteilhaft sein, wenn die durch Verseifung der im erfindungsgemäßen Gemisch enthaltenen Diester der Formel I erhaltenen Carbonsäuren der Summenformel $C_8H_{17}COOH$ 1 bis 85 %, insbesondere 1 bis 50 %, bevorzugt 2 bis 20 %, n-Nonansäure aufweisen.

Die Verseifung der Diester der Formel **I** kann nach üblichen Methoden durch Umsetzung mit alkalischen Medien erfolgen (siehe z. B. Ullmann's Enzyklopädie der Technischen Chemie, 5 Ed. A 10, S. 254 - 260, 1986). Die Bestimmung der Anteile der Carbonsäuren an dem erhaltenen Gemisch, insbesondere des Anteils an 3,5,5-Trimethylhexansäure kann auf übliche Weise z. B. durch gaschromatographische Analysemethoden (GC), bevorzugt nach Derivatisierung zum Methylester oder Silylester, erfolgen.

Besonders bevorzugt weisen die Carbonsäurereste der Summenformel $C_8H_{17}COO$ der im Gemisch enthaltenen Diester einen mittleren Verzweigungsgrad von 0,7 bis 2,0, vorzugsweise von 0,9 bis 1,9, bevorzugt von 1,0 bis 1,8 und besonders bevorzugt von 1,2 bis 1,7 auf. Die Carbonsäurereste sind dabei solche, die auf einem Gemisch von zwei oder mehr Isomeren der Carbonsäure der Summenformel $C_8H_{17}COOH$ basieren.

Die Ermittlung des mittleren Verzweigungsgrades kann, wenn wie beim Isosorbiddiester von Carbonsäuren der Summenformel $C_8H_{17}COOH$ ausschließlich am Ring unsubstituierte Diester vorliegen, durch $^1H$-NMR-Methoden erfolgen. Gemäß der vorliegenden Erfindung erfolgt die Ermittlung des Verzweigungsgrades bevorzugt mit Hilfe der $^1H$-NMR-Spektroskopie an einer Lösung der Diester in Deuterochloroform ($CDCl_3$). Für die Aufnahme der Spektren können zum Beispiel 20 mg Substanz in 0,6 ml $CDCl_3$ (enthaltend 1 Massen-% TMS) gelöst und in ein NMR-Röhrchen mit einem Durchmesser von 5 mm gefüllt werden. Sowohl die zu untersuchende Substanz als auch das verwendete $CDCl_3$ können zunächst über Molekularsieb getrocknet werden, um Verfälschungen der Messwerte durch evtl. vorhandenes Wasser auszuschließen. Die Methode der Bestimmung des Verzweigungsgrades ist gegenüber anderen Methoden zur Charakterisierung von Alkoholresten, wie sie z.B. in WO 03/029339 beschrieben werden vorteilhaft, da Verunreinigungen mit Wasser im Wesentlichen keinen Einfluss auf die Messergebnisse und deren Auswertung haben. Da die Signalgruppe um 2,3 ppm bei z. T. $\alpha$-verzweigten Isononansäuren neben $-CH_2-COOR$ auch -CH-COOR - Gruppen bezeichnet, wird als Referenz die Signalgruppe der veresterten OCH-Gruppen des Isosorbids zwischen 5,3 und 5,5 ppm herangezogen. Die NMR-spektröskopischen Untersuchungen können prinzipiell mit jedem handelsüblichen NMR-Gerät durchgeführt

werden. Für die vorliegende NMR-spektroskopische Untersuchung wurde ein Gerät vom Typ Avance 500 der Firma Bruker eingesetzt. Die Spektren wurden bei einer Temperatur von 303 K mit einem Delay (Verzögerung) von d1 = 5 Sekunden, 32 Scans (Durchgänge), einem 30°C Puls und einer Sweep Width (Spektrale Breite) von 10000 Hz mit einem 5 mm BBO-Probenkopf (broad band observer; Breitbandbeobachtung) aufgenommen. Die Resonanzsignale werden gegen die chemischen Verschiebungen von Tetramethylsilan (TMS = 0 ppm) als internen Standard aufgezeichnet. Mit anderen handelsüblichen NMR-Geräten werden mit den gleichen Betriebsparametern vergleichbare Ergebnisse erhalten.

Die erhabenen $^1$H-NMR-Spektrcn der Gemische von Diestern des Isosorbids weisen im Bereich von 0,5 ppm bis zum Minimum des niedrigsten Tals im Bereich von 0,9 bis 1,1 ppm Resonanzsignale auf, die durch die Signale der Wasserstoffatome der Methylgruppe(n) der Carbonsäuregruppen gebildet werden. Die Signale im Bereich der chemischen Verschiebungen von 3,5 bis 5,5 ppm können den Signalen des Isosorbidgrundkörpers zugeordnet werden, wobei es z. T. zu Überlagerungen der Einzelsignale der insgesamt 8 Protonen kommt (1+1) : 1 :1:(1+1+1):1. Die Quantifizierung erfolgt durch Bestimmung der Fläche unter den jeweiligen Resonanzsignalen, d.h. der vom Signal von der Grundlinie eingeschlossenen Fläche. Handelsübliche NMR-Geräte verfügen über Vorrichtungen zur Integration der Signalfläche. In den vorliegenden NMR-spektroskopischen Untersuchung wurde die Integration mit Hilfe der Software "xwinnmr", Version 3.5 durchgeführt. Anschließend werden die Integralwerte der Signale im Bereich von 0,5 bis zum Minimum des niedrigsten Tals im Bereich von 0,9 bis 1,1 ppm (= $I(CH_3)$) und der Signale im Bereich von 5,0 bis 5,3 ppm (= I(OCH)) ins Verhältnis gesetzt und jeweils durch die Anzahl der entsprechenden Protonen dividiert. Da pro Methylgruppe drei Wasserstoffatome vorhanden sind und jedes Molekül zwei $C_9$-Säurereste enthält, muss die Intensität des $CH_3$-Gruppensignals durch 6 geteilt werden; da pro Molekül 2 veresterte OCH-Gruppen des Isosorbids vorhanden sind, muss dieses Signal durch 2 dividiert werden, um die Anzahl der Methylgruppen pro Isononanoylrest zu erhalten. Da eine lineare primäre Nonansäure, welche nur eine Methyl-Endgruppe aufweist, keine Verzweigung enthält, und demnach einen Verzweigungsgrad von 0 aufweisen muss, muss von diesem Wert noch der Betrag 1 subtrahiert werden

Der Verzweigungsgrad V kann also gemäß folgender Gleichung aus dem gemessenen Intensitätsverhältnis berechnet werden:

$$V = \frac{\frac{I_{CH_3}}{6}}{\frac{I_{OCH}}{2}} - 1$$

V = mittlerer Verzweigungsgrad, d.h. Anzahl der Verzweigungen pro $C_9$-Säurcrest $I(CH_3)$ = Flächenintegral zwischen 0,5 und ca. 1,0 ppm, welches den Methylwasserstoffatomen zugeordnet ist

I(OCH) = Flächenintegral zwischen 5,0 und 5,3 ppm, der veresterten OCH-Gruppen des Isosorbids.

Alternativ kann die Bestimmung des Verzweigungsgrades auch dadurch erfolgen, dass die zur Veresterung eingesetzten Carbonsäuren in die Methylester überführt und dann analog zu dem oben beschriebenen Verfahren die Intensität der zu den Methylgruppen des Alkylrestes gehörenden Signale ($I(CH_3)$) relativ zur Intensität des Methoxysignals der Estergruppe ($I(OCH_3)$) bestimmt wird und die beiden zueinander ins Verhältnis gesetzt werden. Diese Methode hat den Vorteil, dass sie auch bei am Ring substituierten Diestern angewendet werden kann. Der Verzweigungsgrad lässt sich bei dieser Vorgehensweise gemäß folgender Gleichung bestimmen:

$$V = [\, I(CH_3) / I(OCH_3) \,] - 1$$

Damit die Diester der Formel **I**, insbesondere die Isosorbiddiester ein möglichst großes Anwendungsspektrum besitzen, sollten sie bevorzugt bei Raumtemperatur flüssig und möglichst niedrigviskos sein. Bei den Phthalsäureestern sinkt die Viskosität mit zunehmender Linearität des verwendeten Nonylalkohols ($C_9$-Alkohol) bzw. Nonylalkoholgemisches ($C_9$-Alkoholgemisch). Das Di-n-Nonylphthalat weist somit die niedrigste Viskosität auf und kann somit am leichtesten verarbeitet werden. Überraschenderweise wurde gefunden, dass sich bei den Diestern der Formel 1, insbesondere den Isosorbiddiestern ein völlig anderer Sachverhalt darstellt. Wird Isosorbid mit Pelargonsäure (n-Nonansäure) verestert, so ist der entstehende Weichmacher bei Raumtemperatur fest (Mp nach eigenen Messungen mit Differential Scanning Calorimetry DSC bei 27 °C, "Onset") und kann somit beispielsweise für die meisten Plastisolanwendungen nicht ohne vertretbaren Mehraufwand verwendet werden. Weiterhin ist der Diester der ebenfalls kommerziell erhältlichen 3,5,5-Trimethylhexansäure bei Raumtemperatur nur noch sehr eingeschränkt fließfähig (Schmelzpunkt 21,7 °C, "Onset"

bei DSC-Messung). Hingegen sind die erfindungsgemäßen Diester der Formel I, insbesondere Isosorbiddiester, die auf mindestens zwei unterschiedlichen Carbonsäuren der Summenformel $C_8H_{17}COOH$, basieren, gut fließfähig und zeigen, wie in den Beispielen dargestellt, gute anwendungstechnische Eigenschaften. Besonders gut geeignet sind insbesondere solche Diester der Formel **I**, insbesondere Isosorbiddiester, die auf einem Gemisch von Carbonsäuren der Summenformel $C_8H_{17}COOH$, welche aus den Dimeren des 1- bzw. 2-Butens hergestellt wurden, basieren.

Die erfindungsgemäßen Gemische von Diestern der Formel **I** können unterschiedlich zusammengesetzt sein:

a) Das erfindungsgemäße Gemisch kann ausschließlich Diester aufweisen, die alle die gleiche bicyclische Unterstruktur von Formel **I** aufweisen und die einzelnen Diester-Isomeren sich nur durch unterschiedlich strukturierte Carbonsäurereste unterscheiden. Ein solches Gemisch besteht also aus Diester-Isomeren, die alle das gleiche Dianhydrohexitolderivat-Grundgerüst haben. Die Isomerie besteht darin, dass mindestens zwei unterschiedliche $C_9$-Carbonsäurereste vorhanden sind, so dass die einzelnen Diester-Isomere zwei jeweils gleiche oder unterschiedliche $C_9$-Carbonsäurereste aufweisen können. Sind nur zwei unterschiedliche $C_9$-Carbonsäurereste vorhanden, weist die Mischung damit maximal 4, im Fall von Isosorbiddiestern maximal 3, verschiedene Diester-Isomere auf.
b) Das erfindungsgemäße Gemisch kann z. B. mindestens zwei Diester-Isomere mit verschiedenen bicyclischen Unterstrukturen von Formel **I** aufweisen, die sich durch ihre Konfiguration unterscheiden. Ein solches Gemisch kann also aus Diester-Isomeren bestehen, die zwei oder mehr Dianhydrohexitolderivat-Grundgerüste mit unterschiedlicher Konfiguration haben. Es sind wiederum mindestens zwei unterschiedliche $C_9$-Carbonsäurereste vorhanden.
c) Das erfindungsgemäße Gemisch kann z. B. mindestens zwei Diester mit unterschiedlichen Molmassen aufweisen. Ein solches Gemisch kann aus Diestern bestehen, die zwei oder mehr Dianhydrohexitolderivat-Grundgerüste mit unterschiedlicher Molmasse (bedingt durch Substitution des Grundgerüstes) haben. Es sind wiederum mindestens zwei bezüglich ihrer Konstitution unterschiedliche $C_9$-Carbonsäurereste vorhanden.
d) Das erfindungsgemäße Gemisch kann sowohl Diester unterschiedlicher Molmasse als auch Diester-Isomere mit unterschiedlicher Konfiguration der bicyclischen Unterstruktur aufweisen.

Es kann vorteilhaft sein, wenn die im erfindungsgemäßen Gemisch enthaltenen Diester der Formel I solche sind, insbesondere ausschließlich solche sind, bei denen Reste $R^1$ bis $R^8$ jeweils H sind.

Ein besonders bevorzugtes erfindungsgemäßes Gemisch zeichnet sich dadurch aus, dass die enthaltenen Diester der Formel **I** ausschließlich Diester der Formel **Ia**

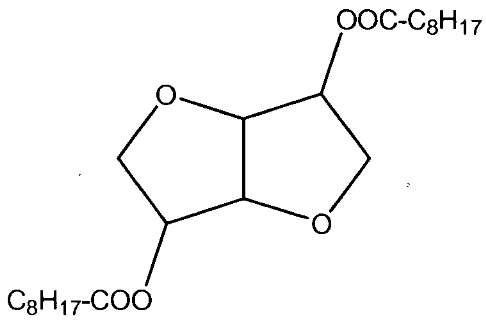

**Ia**

sind, wobei die chiralen C-Atome des bicyclischen Grundgerüstes unabhängig voneinander R oder S konfiguriert sein können. Je nach der relativen Stellung der Säure-Gruppen kann es sich bei den Diestern der Formel **Ia** um die Diester des Isomannids, Isoidids oder Isosorbids handeln. Besonders bevorzugt sind die Diester der Formel **I** ihn dem Gemisch ausschließlich Diester des Isosorbids.

Das erfindungsgemäße Gemisch kann entweder ausschließlich aus den Diestern der Formel I bestehen oder neben diesen zumindest ein Polymer und/oder zumindest einen Weichmacher, der kein Diester der Formel I ist, aufweisen. Die Weichmacher können z. B. ausgewählt sein aus den Citronensäuretrialkylestern, acylierten Citronensäuretrialkylestern, Glycerinestern, Glycoldibenzoaten; Alkylbenzoaten, Dialkyladipaten, Trialkyltrimeilitaten, Dialkylterephthalaten, Dialkylphthalaten oder den Dialkylestern der 1,2-, 1,3- oder 1,4-Cyclohexandicarbonsäuren, wobei die Alkylreste von 4 bis 13, vorzugsweise 5, 6, 7, 8, 9, 10, 11, 12 oder 13 Kohlenstoffatome aufweisen. Die Weichmacher können auch Dianhydröhexitolester, bevorzugt Isosorbiddiester, anderer Carbonsäuren, wie zum Beispiel n- oder iso-Buttersäure, Valeriansäure oder 2-Ethylhexansäure sein.

Polymere, welche in dem erfindungsgemäßen Gemisch enthalten sein können, sind z. B. Polyvinylchlorid (PVC), Polyvinylbutyral (PVB) und die Polyalkylmethacrylate (PAMA). Besonders bevorzugt ist das Polymer Polyvinylchlorid (PVC).

In bevorzugten Gemischen, die Diester der Formel **I** und Polymere aufweisen, beträgt das Massen-Verhältnis von Polymer/Polymeren zu Diester/-n der Formel **1** vorzugsweise von 30 zu 1 bis 1 zu 2,5 und bevorzugt von 20 zu 1 bis 1 zu 2.

In bevorzugten Gemischen, die Diester der Formel **1** und Weichmacher, die kein Diester der Formel I sind, aufweisen, beträgt das Mol-Verhältnis von Weichmachern, insbesondere von Alkylbenzoaten, Dialkyladipaten, Citronensäuretrialkylestern, acylierten Citronensäuretrialkylestern, Trialkyltrimellitaten, Glycoldibenzoaten, Dialkylterephthalaten, Dialkylphthalaten, Dialkanoylestern des Isosorbid und/oder den Dialkylestern der 1,2-, 1,3- oder 1,4-Cyclohexandicarbonsäuren, zu Diester/-n der Formel **I** vorzugsweise von 1 zu 10 bis 10 zu 1 bevorzugt von 1 zu 6 bis 6 zu 1.

Die erfindungsgemäßen Gemische von Diester der Formel **I** bzw. die Diester der Formel **I** selbst können auf verschiedene Weisen hergestellt werden. Vorzugsweise werden die Gemische von Diester der Formel **I** bzw. die Diester der Formel **I** mit dem nachfolgend beschriebenen Verfahren hergestellt.

Das erfindungsgemäße Verfahren zur Herstellung von Diestern der Formel **I**

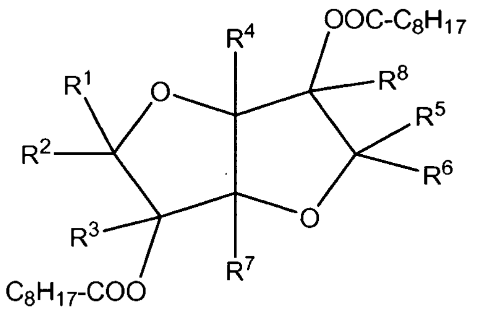

**I**

mit $R^1$ bis $R^8$ = H oder Alkylgruppe mit 1 bis 6 Kohlenstoffatomen, wobei die Reste $R^1$ bis $R^8$ gleich oder unterschiedlich sein können, zeichnet sich dadurch aus, dass ein sechswertiger Alkohol der Formel **II**,

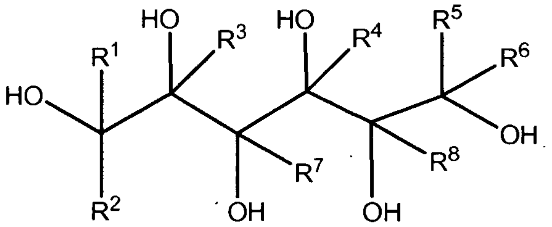

**II**

wobei die Reste $R^1$ bis $R^8$ die in Formel **I** genannte Bedeutung haben, und/oder ein Anhydro- oder Dianhydro-Derivat eines Alkohols der Formel **II** mit einem Gemisch, das zumindest zwei unterschiedliche Carbonsäuren der Summenformel $C_8H_{17}COOH$ aufweist, umgesetzt wird.

Vorzugsweise wird ein Carbonsäuregemisch eingesetzt, welches mindestens zwei Carbonsäuren der Summenformel $C_8H_{17}COOH$ mit unterschiedlicher Konstitutionsformel aufweist, wobei keine der im Gemisch vorhandenen Carbonsäuren einen Anteil von mehr als 95 Mol-%, bevorzugt zumindest 90 Mol-%, aufweist.

Bevorzugt enthalten die im erfindungsgemäßen Verfahren eingesetzten Gemische isomerer Carbonsäuren der Summenformel $C_8H_{17}COOH$ weniger als 10 Mol-%, vorzugsweise weniger als 5 Mol-%, bevorzugt weniger als 1 Mol-% und insbesondere von 0 bis 0,5 Mol-%, bevorzugt weniger als 0,1 Mol-%, insbesondere von 0,0001 bis 0,1 Mol-% und besonders bevorzugt weniger als 0,05 Mol-%, insbesondere von 0,01 bis 0,05 Mol % an 3,5,5-Trimethylhexansäure oder anderen dreifach substituierten Carbonsäuren mit der Summenformel $C_8H_{17}COOH$, insbesondere solchen mit quartären C-Atomen. Die Isomerenverteilungen der isomeren Carbonsäuren in den Gemischen können mit den üblichen, dem Fachmann geläufigen Messmethoden wie NMR-Spektroskopie, GC- oder GC/MS-Spektroskopie, bevorzugt nach überführung in die Silyl- oder Methylester, ermittelt werden.

Besonders bevorzugt wird in dem erfindungsgemäßen Verfahren ein Gemisch von isomeren Carbonsäuren der Summenformel $C_8H_{17}COOH$ eingesetzt, das einen mittleren Verzweigungsrad von 0,7 bis 2,0, vorzugsweise von 1,0 bis 1,9, bevorzugt von 1,1 bis 1,8 und besonders bevorzugt von 1,1 bis 1,7 aufweist. n-Nonansäure weist beispielsweise einen Verzweigungsgrad von 0 auf, 3,5,5-Trimethylhexansäure weist einen Verzweigungsgrad von 3 auf. Der

Verzweigungsgrad des Gemisches ergibt sich aus der Summe der Verzweigungsgrade der Einzelkomponenten multipliziert mit dem jeweiligen Massen- oder Mol-Anteil der Einzelkomponente geteilt durch die Summe der Anteile aller Einzelkomponenten.

Der Verzweigungsgrad für Gemische kann im einfachsten Fall, durch direktes Bestimmen der Anteile der Einzelkomponenten bestimmt werden. Ist eine solche Bestimmung nicht möglich, so kann der Verzweigungsgrad für Gemische isomerer Nonansäuren z. B. mittels $^1$H-NMR analog zur oben beschriebenen Methode bestimmt werden.

**Herstellung der Nonansäure**

Prinzipiell können alle technischen Gemische von Carbonsäuren mit der Summenformel $C_8H_{17}COOH$, die zumindest zwei unterschiedliche Konstitutions-Isomere aufweisen, eingesetzt werden. Vorzugsweise werden solche Gemische isomerer Carbonsäuren mit der Summenformel $C_8H_{17}COOH$ eingesetzt, die hinsichtlich des Anteils der verschiedenen Isomeren, des mittleren Verzweigungsgrads und/oder des Gehalts an 3,3,5-Trimethylhexansäure in den oben angegebenen Bereichen liegen.

Die im erfindungsgemäßen Verfahren eingesetzten Gemische isomerer Carbonsäuren mit der Summenformel $C_8H_{17}COOH$ (nachfolgend isomere Nonansäuren genannt) können beispielsweise durch Hydroformylierung von Octenen, die wiederum auf unterschiedliche Art erzeugt werden können, und anschließende Oxidation hergestellt werden.

Als Rohstoff zur Herstellung der Octene können im Allgemeinen technische $C_4$-Ströme eingesetzt werden, die zunächst alle isomeren $C_4$-Olefine neben den gesättigten Butanen und gegebenenfalls Verunreinigungen wie $C_3$- und $C_5$-Olefinen und acetylenischen Verbindungen enthalten. Durch Oligomerisierung der in den $C_4$-Strömen enthaltenen Olefine erhält man vorwiegend isomere Octengemische neben höheren Oligomeren wie $C_{12}$- und $C_{16}$-Olefingemischen. Diese Octengemische können, gegebenenfalls nach einer destillativen Abtrennung der $C_{12}$- und $C_{16}$-Olefine, zu den entsprechenden Aldehyden hydroformyliert werden und können anschließend zur Carbonsäure oxidiert werden. Die Zusammensetzung, d. h. die Isomerenverteilung der technischen Nonansäuregemische ist abhängig vom Ausgangsmaterial und von den Oligomerisierungs-, Oxidations- und Hydroformylierungsverfahren.

Als Octen-Gemische können z. B. auch solche eingesetzt werden, die über das sogenannte Polygas-Verfahren erhalten werden, bei dem eine Oligomerisierung von $C_3$-/$C_4$-Mischungen an einem festen sauren Katalysator, vorzugsweise an einem festen Phosphorsäure-Katalysator (SPA-Verfahren) durchgeführt wird. Dieses Verfahren wird unter anderem in den Dokumenten US 6,284,938, US 6,080,903, US 6,072,093, US 6,025,533, US 5,990,367, US 5,895,830, US 5,856,604, US 5,847,252 und US 5,081,086 beschrieben. Werden ausschließlich auf diese Weise erhaltene Olefingemische hydroformyliert, so werden in der Regel auch noch Anteile von Octanalen und Decanalen erhalten, so dass hier die mittlere Kettenlänge von 9 Kohlenstoffatomen abweichen kann. Nach der Oxidation wird also ein isomere Nonansäuren aufweisendes Gemisch erhalten, welches auch noch Isomere der Octan- bzw. Decansäure aufweisen kann. Auf die Bestimmung des Verzweigungsgrads V gemäß der oben genannten Methode hat dies aber keine Auswirkung.

Weiterhin können auch Octene aus der Ethylen-Oligiomerisierung vorteilhaft verwendet werden.

Besonders bevorzugte, im erfindungsgemäßen Verfahren einsetzbare Gemische isomerer Nonansäuren sind solche, die erhältlich sind durch Hydroformylierung von isomeren Octenen und anschließende Oxidation der entstehenden Aldehyde und ggf. entstehender Alkohole, wobei das Gemisch isomerer Octene durch Inkontaktbringen eines Butene aufweisenden Kohlenwasserstoffgemisches, welches einen Anteil an Isobuten von vorzugsweise kleiner 20 Massen-%, bevorzugt kleiner 10 Massen-%, besonders bevorzugt kleiner 5 Massen-%, ganz besonders bevorzugt kleiner 3 Massen-%, insbesondere bevorzugt kleiner 1 Massen-%, vorzugsweise zwischen 0,01 und 1 Massen-% und besonders bevorzugt zwischen 0,05 und 0,5 Massen-% bezogen auf die Butene aufweist, mit einem Oligomerisierungskatalysator, insbesondere mit einem Nickeloxid enthaltenden Katalysator, erhalten wird. Die Herstellung von isomeren Octenen durch Oligomerisierung von im Wesentlichem linearen Butenen an Nickelträgerkatalysatoren ist z. B. als OCTOL-Prozess bekannt, der z. B. in EP 0 395 857 oder EP 1 029 839 beschrieben wird. In Varianten zum OCTOL-Prozess werden z. B. Ti oder Zr aufweisende Katalysatoren eingesetzt. Solche alternativen Varianten und insbesondere die Katalysatoren werden z. B. in EP 1 171 413 beschrieben. Wie bereits oben beschrieben, können die so erhaltenen Octene von den höheren Olefinen, also den $C_{12}$-, $C_{16}$-, $C_{20}$- etc. Olefinen z. B. destillativ abgetrennt werden.

**Hydroformylierung**

Die z. B. wie oben beschrieben hergestellten Octene oder Gemische isomerer Octene, werden einer Hydroformylierung zugeführt. Die Hydroformylierung kann in Gegenwart von modifizierten oder unmodifizierten Kobalt- oder Rhodiumkatalysatoren erfolgen. Vorzugsweise erfolgt die Hydroformylierung in Gegenwart von unmodifizierten Kobaltverbindungen. Geeignete Hydroformylierungs-Verfahren sind z. B. aus EP 0 850 905 und EP 1 172 349 bekannt. In der Regel entsteht auf diese Weise ein Gemisch aus im Wesentlichen isomeren Nonanalen, eventuell noch nicht umgesetzten Octenen sowie den entsprechenden, durch Hydrierung (Folgereaktion) entstehenden Gemischen aus isomeren Nonanolen und Octanen.

Die Hydroformylierung kann auch in Gegenwart von Rhodiumkatalysatoren erfolgen. Solche Hydroformylierungsverfahren sind allgemein, so z. B. aus EP 0 213 639, EP 1 201 675, WO 03/16320, WO 03/16321, WO 2005/090276 und den dort zitierten Schriften bekannt. Spezielle Verfahren zur Hydroformylierung, die zur Herstellung von im erfindungsgemäßen Verfahren einsetzbaren Gemischen isomerer Nonansäuren ebenfalls geeignet sind, werden z. B. in WO 2004/020380 oder DE 103 27 435 beschrieben. Die dort beschriebenen Verfahren werden in Gegenwart von cyclischen Kohlensäureestern durchgeführt.

Es kann auch vorteilhaft sein, das Gemisch isomerer Octene vor der Zuführung zur Hydroformylierung zunächst wie in EP 1 172 349 beschrieben zu fraktionieren. Auf diese Weise ist es möglich, Octenfraktionen zu erhalten, die besonders gut zur Herstellung von im erfindungsgemäßen Verfahren einsetzbaren Gemischen isomerer Nonansäuren geeignet sind. Aus den Fraktionen kann dann auf relativ einfache Weise durch Mischen von geeigneten Fraktionen ein Gemisch von isomeren Octenen erhalten werden, welches zur Herstellung von Gemischen von isomeren Nonansäuren zum Einsatz im erfindungsgemäßen Verfahren geeignet ist.

Optional kann und bevorzugt wird das aus der Hydroformylierung erhaltene Reaktionsgemisch fraktioniert und so die für die Oxidation bestimmte Nonanalfraktion aufkonzentriert. Insbesondere dann, wenn in der für die Oxidation vorgesehenen Mischung aus Nonanalen noch ein erhöhter Anteil an Nonanolen vorliegt, empfiehlt sich die destillative Aufreinigung.

Die Hydroformylierung der Octengemische kann ein- oder mehrstufig, optional mit Abtrennung der nicht reagierten Octene nach jeder Stufe, durchgeführt werden.

## Oxidation

Die Oxidation des $C_9$-Aldeyhds oder der in einem Gemisch vorhandenen zwei oder mehr isomeren $C_9$-Aldehyde zu den entsprechenden Carbonsäuren kann in an sich bekannter Weise erfolgen. Als Oxidationsmittel können z. B. Sauerstoff, Luft oder andere sauerstoffhaltige Gase eingesetzt werden. Die Oxidation kann unkatalysiert oder katalysiert durchgeführt werden. Im letzteren Falle kann es vorteilhaft sein, wenn Verbindungen von Übergangsmetallen, insbesondere Kobalt als Katalysatoren verwendet werden. Die Oxidation der Aldehyde kann bei Normaldruck oder erhöhtem Druck (1 bis 10 bar, bevorzugt 1,1 bis 5 bar) durchgeführt werden. Die Reaktionstemperaturen liegen im Bereich von 30 °C bis 150 °C, bevorzugt zwischen 40 und 90 °C, besonders bevorzugt zwischen 50 und 80 °C. Die Reaktionszeiten können den genannten Oxidationsbedingungen angepasst werden und können wenige Minuten bis mehrere Stunden betragen.

Die Carbonsäure/-n kann/können aus dem Oxidationsgemisch durch Destillation bei Normaldruck oder bei vermindertem Druck gewonnen werden. Gegebenenfalls können die Carbonsäuregemische in Fraktionen mit unterschiedlichen Carbonsäuren aufgetrennt werden. Auch auf diese Art können dann nochmals Nonansäurefraktionen erhalten werden, die für die Herstellung der erfindungsgemäßen Dianhydrohexitolester, bevorzugt Isosorbiddiester der Formel 1, besonders geeignet sind. Durch diese Fraktionierung und ein anschließendes Mischen der Fraktionen miteinander oder auch mit anderen Carbonsäuregemischen ist es insbesondere möglich, Carbonsäuregemische zusammenzustellen, die oben genannten bevorzugten Anteile an bestimmten Komponenten bzw. einen bestimmten Verzweigungsgrad aufweisen. Auf diese einfache Weise lassen sich Gemische von Diestern der Formel **I** erhalten, die die gewünschten Eigenschaften aufweisen.

Im erfindungsgemäßen Verfahren kann aber auch als Gemisch isomerer Nonansäuren ein Gemisch eingesetzt werden, das durch Mischen von isomerenreinen Nonansäuren und/oder Fraktionen von mehreren isomeren Nonansäuren erhältlich ist. Mindestens zwei isomerenreine Nonansäuren sind kommerziell erhältlich, nämlich die n-Nonansäure (Pelargonsäure) und die 3,5,5-Trimethylhexansäure (CAS-Nummer 3302-10-1). Ebenso sind Nonansäure-Gemische oder -Fraktionen kommerziell erhältlich, die nicht die für das erfindungsgemäße Verfahren bevorzugten Eigenschaften aufweisen. Hierbei handelt es sich im Wesentlichen um Gemische von mehrfach verzweigten Isononansäuren mit einem hohen (93 bis 95 %) Anteil an 3,5,5-Trimethylhexansäure (*CAS-Nummer 26896-18-4, Fa. Celanese*). Durch einfaches Mischen von solchen isomerenreinen Nonansäuren mit anderen isomerenreinen Nonansäuren oder mit Nonansäure-Gemischen lassen sich Gemische von Nonansäuren herstellen, die bei der Veresterung zu Diestern der Formel **I** mit den gewünschten Eigenschaften führen. Insbesondere ist es durch ein solches einfaches Mischen möglich, Gemische von Nonansäuren zu erhalten, die den gewünschten Anteil an 3,5,5-Trimethylhexanol und an sonstigen Komponenten aufweisen.

## Alkoholeinsatzstoff

Die in dem erfindungsgemäßen Verfahren eingesetzte Alkoholkomponente ist ausgewählt aus einem Alkohol der Formel **II** oder einem Dianhydro- oder Monoanhydro-Derivat dieses Alkohols. Vorzugsweise wird als Alkohol der Formel **II** ein Alkohol, bei dem die Reste $R^1$ bis $R^8$ jeweils H sind, oder ein Dianhydro- oder Monoanhydro-Derivat dieses

Alkohols eingesetzt. Besonders bevorzugt sind aus dieser Gruppe die Hexitole Sorbitol, Mannitol und Iditol, ganz besonders bevorzugt ist das Sorbitol. Es können auch Gemische der oben genannten Verbindungen eingesetzt werden.

Bevorzugt wird als Dianhydro-Derivat eines Alkohols der Formel **II** Isosorbid oder eines seiner Konfigurationsisomere Isomannid oder Isoidid **IIa**

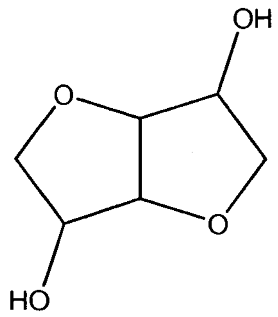

**IIa**

eingesetzt, welches durch zweifache Dehydratisierung (intramolekulare Veretherung) aus dem Alkohol der Formel **II**, bei dem alle Rest $R^1$ bis $R^8$ jeweils H sind (Sorbitol bzw. Mannitol oder Iditol), oder aus dem entsprechenden Monoanhydro-Derivat (Sorbitan bzw. Mannitan oder Iditan) der Formel **IIb**

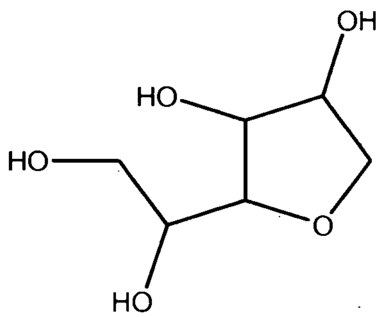

**IIb**

durch einfache Dehydratisierung erhalten werden kann. Besonders bevorzugt wird als Dianhydro-Derivat eines Alkohols der Formel **II** Isosorbid eingesetzt.

Insbesondere Sorbitol ist in Mengen von mehreren hunderttausend Jahrestonnen kommerziell erhältlich, nennenswerte Isosorbid-Kapazitäten sind von mehreren Herstellern angekündigt, so dass die Rohstoffversorgung zumindest mittelfristig gewährleistet ist.

Die Herstellung der erfindungsgemäßen Diester der Formel **I** kann formal auf verschiedene Arten erfolgen, auf die im Folgenden detailliert eingegangen wird.

Zum einen kann die Diester-Herstellung ausgehend vom Dianhydroderivat des Alkohols der Formel I durch Veresterung mit einem Gemisch von zumindest zwei isomeren Nonansäuren erfolgen. Die Veresterung von Dianhydrohexitolen, insbesondere von Isosorbid, mit verschiedenen aliphatischen Carbonsäuren ist in der Literatur verschiedentlich ausführlich beschrieben. Im Allgemeinen wird das Dianhydrohexitol mit der Carbonsäure in Gegenwart eines Katalysators zum entsprechenden Diester des Dianhydrohexitols umgesetzt. Vorzugsweise erfolgt die Umsetzung mit einem Überschuss an Carbonsäure (d. h. mehr als zwei Moläquivalenten), vorzugsweise mit einem molaren Überschuss von 10 bis 100 %, bevorzugt von 20 bis 50 %. Zur Abtrennung des durch die Veresterung gebildeten Wassers aus dem Reaktionsgemisch können verschiedene Verfahren angewendet werden. Das Wasser kann z. B. durch einen durch das Reaktionsgemisch geleiteten Inertgasstrom ausgetrieben oder mittels Vakuum entfernt werden. Weiterhin kann Wasser durch azeotrope Destillation abgetrennt werden, entweder durch Verwendung eines Schleppmittels wie Toluol, Benzol, Xylol oder Cyclohexan oder die Carbonsäure selbst dient als Schleppmittel und die abdestillierte Menge wird ganz oder teilweise durch die Carbonsäure ersetzt. Eine Übersicht über die in der Literatur beschriebenen Verfahren fmdet sich in WO 2006/103338. Dort wird neben einer Beschreibung des Stands der Technik auch ein Verfahren zur Herstellung von Dianhydrohexitoldiestern beschrieben.

Zum anderen kann die Herstellung der erfindungsgemäßen Diester der Formel **I** ausgehend vom Hexitol der Formel **II** durch eine Reaktionssequenz, bestehend aus einer zweifachen intramolekularen Wasserabspaltung zum Dianhydroderivat mit nachfolgender oder zeitgleicher Veresterung oder ausgehend vom Monoanhydroderivat des Hexitols der Formel **II**, bestehend aus einer einfachen intramolekularen Wasserabspaltung zum Dianhydroderivat mit nachfolgender oder zeitgleicher Veresterung durchgeführt werden. Die beiden Einzelschritte der Reaktion (Veresterung und ein- oder mehrfache Wasserabspaltung) können getrennt oder auch als so genannte Eintopfreaktion durchgeführt werden. Eine Beschreibung der Durchführung findet sich beispielsweise in WO 01/83488.

**Dehydratisierung (Intramolekulare Veretherung)**

Wird das Mono-Anhydro-Derivat eines Alkohols der Formel **II** eingesetzt, so wird dieses formal in einer vorgelagerten oder zeitgleich stattfindenden intramolekularen Wasserabspaltung in das Dianhydro-Derivat überfährt. Wird ein sechswertiger Alkohol der Formel **II** eingesetzt, wird dieser formal in einer vorgelagerten oder zeitgleich stattfindenden doppelten oder einfachen intramolekularen Wasserabspaltung in das Dianhydro- Derivat bzw. das Mono-Anhydro-Derivat überführt, wobei letzteres in einer weiteren intramolekularen Wasserabspaltung ebenfalls in das Dianhydro-Derivat überführt wird.

Die Dehydratisierung (Wasserabspaltung) des Alkohols der Formel **II** oder des entsprechenden Anhydro-Derivats zum Dianhydro-Derivat gemäß Formel **IIc**

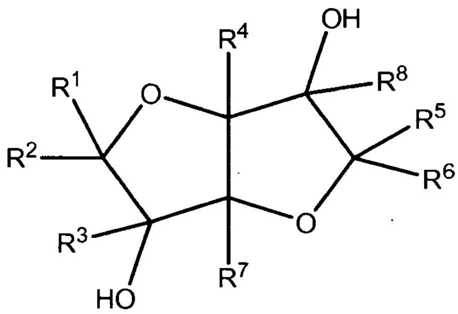

**IIc**

mit $R^1$ bis $R^8$ wie in Formel I definiert, kann in einer Ausführungsform des erfindungsgemäßen Verfahrens in einem separaten Schritt vor der Veresterung durchgeführt werden. Das hierbei erhältliche Reaktionsgemisch, das im Wesentlichen das Dianhydro- und Monoanhydroderivat des Hexitols zusammen mit Nebenprodukten enthält, wird dann entweder direkt der Veresterung zugeführt oder optional zunächst aufgearbeitet, wobei beispielsweise der Katalysator für die Dehydratisierung abgetrennt und durch Reinigungsschritte wie Destillation, Kristallisation, Waschen, Bleichen etc. die Reinheit des für den nächsten Reaktionsschritt benötigten Dianhydrohexitols erhöht werden kann.

Die Dehydratisierung (intramolekulare Veretherung) wird bei dieser Ausführungsform des erfindungsgemäßen Verfahrens vorzugsweise bei einer Temperatur von 100 bis 200 °C, bevorzugt von 110 bis 180 °C, wenn Sorbitol oder dessen Monoanhydro-Derivat dehydratisiert werden sollen, besonders bevorzugt bei einer Temperatur von 120 bis 150 °C bei Normaldruck oder leichtem Vakuum durchgeführt. Die Dehydratisierung wird bei dieser Ausführungsform bevorzugt in Gegenwart eines Katalysators durchgeführt. Falls das Dehydratisierungsgemisch direkt weiter verestert wird, wird besonders bevorzugt ein Katalysator eingesetzt, der mit dem in der Veresterung eingesetzten Katalysator identisch ist. Für diesen Zweck sind nach WO 01/83488 makroporöse saure Ionentauscher besonders geeignet.

In vielen Fällen kann es sich aber aus kinetischen Gründen als vorteilhaft erweisen, das Gemisch isomerer Nonansäuren erst dann dem Reaktionsgemisch zuzugeben, wenn der Alkohol der Formel II bereits überwiegend zum Dianhydroderivat abreagiert hat und auch nur noch wenig Monoanhydroderivat (< 10 %, messbar über GC) zugegen ist. Ansonsten verläuft die zweite Wasserabspaltung vom Monoanhydrohexitol zum Dianhydrohexitol in Konkurrenz zur Veresterung des Monoanhydroderivates ab, was zu geringeren Ausbeuten an Diisononanoylestem des Dianhydrohexitols führt. Die entsprechenden Zeiten, zu denen die Carbonsäure dann bevorzugt zugegeben wird, können über Vorversuche leicht ermittelt werden. Diese Zeiten hängen u. a. von Art und Menge des Katalysators und von der Temperatur ab.

Bei der Dehydratisierung kann es vorteilhaft sein, wenn während des Verfahrens gebildetes Wasser durch Durchleiten eines Gases, insbesondere eines Inertgases durch das Reaktionsgemisch entfernt wird. Als Inertgas kann z. B. Stickstoff eingesetzt werden.

Es ist aber auch möglich, während der Dehydratisierung gebildetes Wasser durch Destillation aus dem Reaktionsgemisch zu entfernen. Bevorzugt wird diese Destillation unter reduziertem Druck durchgeführt.

In einer bevorzugten Ausführungsform des erfindungsgemäßen Verfahrens wird bei Einsatz eines Alkohols

der Formel **II** oder des Monoanhydro-Dcrivats des Alkohols, insbesondere von Sorbitol oder Sorbitan, die Dehydratisierung und die Veresterung in einem Arbeitsgang durchgeführt. In diesem Fall kann das Gemisch isomerer Nonansäuren bereits von Anfang an dem Reaktionsgemisch zugesetzt werden. Bei der Auswahl der Katalysatoren für die Sequenz Dehydratisierung und Veresterung sollte darauf geachtet werden, dass diese eine hohe Selektivität hin zum Diester des Dianhydroderivates aufweisen, da andernfalls in eventuell (unerwünscht) hohen Anteilen die Mono-, Di-, Tri- oder Tetraester des MonoanhydroDerivates des Alkohols der Formel **II** entstehen können. Besonders gut geeignete Katalysatoren sind in der Regel starke Brönstedt-Säuren, wie Schwefelsäure oder die bereits erwähnten makroporösen sauren Ionentauscherharze. Reine Lewis-Säuren wie zum Beispiel Tetrabutyltitanat eignen sich oft nicht für die Dehydratisierungsstufe.

## Veresterung

Die Veresterung kann auf bekannte Weise z. B. durch Reaktion von Alkoholen der Formel **II** bzw. dessen Monoanhydro- oder Dianhydro-Derivats mit einem geeigneten Gemisch isomerer Nonansäuren, erfolgen. Die Veresterung kann autokatalysiert oder katalysiert durchgeführt werden. Vorzugsweise erfolgt die Veresterung in Gegenwart eines Katalysators. Prinzipiell sind alle bekannten Veresterungsverfahren in dem erfindungsgemäßen Verfahren einsetzbar. Vorzugsweise erfolgt der Veresterungsschritt allerdings nach einem Verfahren, bei dem das entstehende Reaktionswasser durch azeotrope Destillation mit der Carbonsäure entfernt wird und die durch die azeotrope Destillation aus der Reaktion entfernte Flüssigkeitsmenge vollständig oder teilweise mit dem Gemisch der isomeren Nonansäuren wieder ergänzt wird. Als Flüssigkeitsmenge wird im Folgenden das durch azeotrope Destillation aus der Reaktion entfernte Flüssigkeitsvolumen, hauptsächlich bestehend aus Reaktionswasser und isomeren Nonansäuren, bezeichnet. Ein vollständiger Ersatz der entfernten Flüssigkeitsmenge ist bevorzugt. Dies kann z. B. durch eine standgeregelte Einspeisung eines Gemisches von isomeren Nonansäuren in den Reaktor erfolgen.

Aus technischen Gründen kann ein vollständiger Ersatz der entfernten Flüssigkeitsmenge nicht oder nur schwer realisierbar sein. In diesen Fällen wird die entfernte Flüssigkeitsmenge nur teilweise, z. B. nur das Gemisch der isomeren Nonansäuren, nicht jedoch das entfernte Reaktionswasser, in jedem Fall aber zu mehr als 90 %, bevorzugt 95 bis 98 % wieder ersetzt. Es kann auch erforderlich sein, mehr als die abdestillierte Flüssigkeitsmenge in den Reaktor zurückzuführen, d. h. neben der entfernten Säuremenge wird das Reaktionswasser ersetzt und darüber hinaus weitere Säure zugegeben. In dieser Ausführungsform der Veresterung wird 110 bis 100 %, bevorzugt 105 bis 100 % der entfernten Flüssigkeitsmenge durch Säure ersetzt.

Diese Ausführungsform der Veresterung hat den Vorteil, dass im Vergleich zu bekannten diskontinuierlichen Verfahren die Reaktionsgeschwindigkeit erhöht wird. Dadurch kann die Taktzeit verkürzt werden, wodurch eine höhere Raum-Zeit-Ausbeute erreicht wind.

Die Veresterung wird vorzugsweise in einem Reaktionsgefäß durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Ist ein Einsatzstoff bei der Einfülltemperatur fest, ist es zweckmäßig, die flüssige Einsatzkomponente vorzulegen. Feste Einsatzstoffe können als Pulver, Granulat, Kristallisat oder Schmelze eingespeist werden. Um die Chargenzeit zu verkürzen, ist es ratsam, während des Einfüllens mit dem Aufheizen zu beginnen. Der Katalysator kann in reiner Form oder als Lösung, bevorzugt gelöst in einem der Einsatzstoffe, zu Beginn oder erst nach Erreichen der Reaktionstemperatur eingebracht werden.

Das umzusetzende Gemisch isomerer Nonansäuren, das auch als Schleppmittel dient, kann im stöchiometrischen Überschuss eingesetzt werden. Bevorzugt wird ein Überschuss von 5 bis 50 %, besonders bevorzugt 10 bis 30 % eingesetzt.

Bezüglich der Durchführung der Veresterung (falls vom Dianhydro-Derivat ausgegangen wird) oder der Sequenz aus Dehydratisierung und Veresterung (falls vom sechswertigen Alkohol der Formel II oder dessen Monoanhydro-Derivat ausgegangen wird) sei insbesondere auf EP 1 278 752 (WO 01/83488) verwiesen. Die dort beschriebenen Verfahren können besonders vorteilhaft zur Synthese der erfindungsgemäßen Ester der Formel I a eingesetzt werden.

Die Veresterung ausgehend von reinem Dianhydroderivat wird vorzugsweise an einem makroporösen sauren Ionenaustauscherharz als Veresterungskatalysator durchgeführt. Die Reaktionstemperaturen bei der Veresterung werden in der Regel von der Temperaturstabilität dieser Harze nach oben begrenzt. Je nach Vernetzungsgrad können diese Harze daher bei maximalen Temperaturen von 100 bis 190 °C verwendet werden. Die entsprechenden Informationen werden vom Hersteller mitgeteilt.

Eine im erfindungsgemäßen Verfahren bevorzugt eingesetzte Gruppe von sauren Ionenaustauscherharzen, sind insbesondere solche mit Sulfonsäuregruppen. Geeignete Ionenaustauscherharze können beispielsweise solche sein, die durch Sulfonierung von PhenoUAldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung

von Ionenaustauscherharzen mit Sulfonsäuregruppen verwendet.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können die Ionenaustauscherharze in ihrer H-Form eingesetzt werden. Stark saure makroporöse Harze des Styrol-Divinylbenzol-Typs werden u. a. unter folgenden Handelsnamen verkauft: Amberlyst 15, Amberlyst 35, Amberlyst 70.

Das Porenvolumen der bevorzugt eingesetzten Ionenaustauscherharze beträgt vorzugsweise von 0,3 bis 0,9 ml/g, insbesondere von 0,5 bis 0,9 ml/g. Die Korngröße der bevorzugt eingesetzten Harze beträgt vorzugsweise von 0,3 mm bis 1,5 mm, insbesondere von 0,5 mm bis 1,25 mm. Die Korngrößenverteilung kann enger oder weiter gewählt werden. Besonders bevorzugt werden Ionenaustauscherharze mit sehr einheitlicher Korngröße eingesetzt. Die Kapazität der bevorzugt eingesetzten Ionenaustauscherharze beträgt, bezogen auf die Lieferform, vorzugsweise von 0,7 bis 2,0 eq/1, insbesondere von 1,1 bis 2,0 eq/1, bzw. vorzugsweise von 0,5 bis 5,5 mol/kg, insbesondere von 0,8 bis 5,5 mol/kg (Die Angaben zur Kapazität in mol/kg beziehen sich auf das jeweils bis zur Gewichtskonstanz im warmen Stickstoffstrom bei z. B. 105 °C getrocknete Ionentauscherharz.).

Neben den Ionenaustauscherharzen können zur Herstellung der Diester ausgehend von Dianhydrohexitol auch zahlreiche andere Katalysatoren eingesetzt werden. Solche Veresterungskatalysatoren können Säuren, wie beispielsweise Schwefelsäure, Methansulfonsäure oder p-Toluolsulfonsäure, oder metallhaltige Katalysatoren sein. Beispielhafte Vertreter für besonders bevorzugt eingesetzte Metallkatalysatoren sind Titansäureester wie Tetraisopropylorthotitanat oder Tetrabutylorthotitanat sowie Zirkoniumester wie Tetrabutylzirkonat. Die Metallkatalysatoren sind im Vergleich zu den Katalysatoren auf Basis von Protonensäuren Hochtemperaturkatalysatoren, die ihre volle Aktivität oft erst bei Temperaturen oberhalb 180 °C erreichen.

Die Katalysatorkonzentration hängt von der Art des Katalysators ab. Bei den bevorzugt eingesetzten Titanverbindungen beträgt diese 0,005 bis 1,0 Massen-% bezogen auf das Reaktionsgemisch, insbesondere 0,01 bis 0,3 Massen-%.

Die Reaktionstemperaturen liegen bei Verwendung von Titankatalysatoren zwischen 160 °C und 260 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise die Bildung farbiger Nebenprodukte. Es ist zur Entfernung des Reaktionswassers erforderlich, dass das Gemisch isomerer Nonansäuren aus dem Reaktionsgemisch abdestillieren kann. Die gewünschte Temperatur oder der gewünschte Temperaturbereich kann durch den Druck im Reaktionsgefäß eingestellt werden. Im Falle der Umsetzung von Isosorbid mit dem Gemisch isomerer Nonansäuren unter Verwendung von Tetrabutyltitanat als Katalysator hat sich ein Temperaturbereich von 180 bis 260 °C, bevorzugt 210 bis 250 °C, als vorteilhaft dargestellt.

Die in die Reaktion zurückzuführende Flüssigkeitsmenge kann teilweise oder vollständig aus isomeren Nonansäuren bestehen, die durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frische isomere Nonansäuren, d. h. aus einem Vorratsgefäß bereitstehende isomere Nonansäuren zu ersetzen. In anderen Ausführungsformen der Veresterung wird die abgetrennte Flüssigkeit zu den isomeren Nonansäuren aufgearbeitet.

Nach Beendigung der Reaktion enthält das Reaktionsgemisch, das im Wesentlichen aus Vollester (Zielprodukt) und überschüssiger Carbonsäure besteht, neben dem Katalysator und/oder dessen Folgeprodukten geringe Mengen an Dianhydrohexitol-Monoester. Daneben können weitere durch Parallel- und/oder Folgereaktionen entstandenen Nebenprodukte, insbesondere die Mono-, Di-, Tri- und Tetraester des Monoanhydroderivates des sechswertigen Alkohols nach Formel II vorhanden sein. In der Regel sind die Rohproduktgemische gelb bis dunkelbraun gefärbt und bedürfen daher für technische Einsetzbarkeit einer unter Umständen aufwändigen Reinigung. Diese kann bei Verwendung von Katalysatorsystemen, wie sie beispielsweise in WO06/103338 beschrieben sind, auch einfacher werden.

Zur Aufarbeitung dieser Esterrohgemische wird der größte Teil des überschüssigen Carbonsäuregemisches mittels Vakuumdestillation und einer optionalen Wasserdampfdestillation, insbesondere im Temperaturbereich von 120 bis 225 °C, entfernt., Danach schließen sich die üblichen Schritte zur Neutralisation, Reinigung, Bleichung und Filtration des Rohproduktes an, die je nach Intensität der Verfärbung bzw. Anteil an wasserlöslichen Nebenprodukten zu unterschiedlichen Zeitpunkten und unterschiedlicher Intensität durchgeführt werden.

Die Neutralisation der sauren Stoffe, wie Carbonsäuren, oder gegebenenfalls der sauren Katalysatoren, kann durch Zugabe von basisch wirkenden Verbindungen der Alkali- und Erdalkalimetalle erfolgen. Diese können in Form ihrer Carbonate, Hydrogencarbonate oder Hydroxide eingesetzt werden. Das Neutralisationsmittel kann in fester Form oder bevorzugt als Lösung, insbesondere als wässrige Lösung eingesetzt werden. Die Neutralisation wird bevorzugt nach der Abdestillation der Hauptmenge der überschüssigen Carbonsäure durchgeführt werden.

In den meisten Fällen ist es ratsam, das neutralisierte Rohprodukt ein oder mehrmals mit Wasser oder Salzlösung zu waschen um wasserlösliche Nebenbestandteile abtrennen zu können.

Die Aufhellung des Rohproduktes kann zum Einen adsorptiv an Feststoffen mit großen Oberflächen wie Aktivkohle aber auch speziellen polymeren Adsorberharzen, beispielsweise auf Basis von Styrol und Divinylbenzol, vorgenommen werden. Alternativ bietet sich die Bleichung mit Wasserstoffperoxid oder Ozon an. Welche der Varianten zur Aufhellung gewählt werden, kann durch Vorversuche ermittelt werden. Gegebenfalls können auch zwei oder mehrere dieser Methoden kombiniert werden.

Nach Abschluss der Aufreinigung wird das Produkt bei erhöhter Temperatur im Vakuum getrocknet und anschließend filtriert.

Weitere Details zu geeigneten Veresterungsverfahren, die in dem erfindungsgemäßen Verfahren als Veresterungsschritt eingesetzt werden können, können z. B. EP 1 186 593 und EP 1 300 388 entnommen werden.

Die erfindungsgemäßen Gemische, die Ester der Formel I enthalten oder aus diesen bestehen, können in Farben, Tinten oder Lacken, in Plastisolen, Klebstoffen oder Klebstoffkomponenten, in Dichtungsmassen, als Weichmacher in Kunststoffen oder Kunststoffkomponenten, als Lösemittel, als Schmierölkomponente, als Kühlflüssigkeit oder Bohrflüssigkeit oder Bestandteil davon oder als Hilfsmittel bei der Metallverarbeitung verwendet werden. Bevorzugte Plastisole sind dabei insbesondere PVC-oder PAMA-Plastisole. Bevorzugte Kunststoffe sind dabei insbesondere Polyvinylchlorid (PVC), Polyvinylbutyral (PVB), Homo- und Copolymere auf Basis von Ethylen, Propylen, Butadien, Vinylacetat, Celluloseacetat, Glycidylacrylat, Glycidylmethacrylat, Methacrylaten, Acrylaten, Acrylaten mit am Sauerstoffatom der Estergruppe gebundenen Alkylresten von verzweigten oder unverzweigten Alkoholen mit einem bis zehn Kohlenstoffatomen, Styrol, Acrylnitril, Homo- oder Copolymere von cyclischen Olefinen.

Als Vertreter der obigen Gruppen seien beispielsweise folgende Kunststoffe genannt: Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, Polymethacrylat, Polymethylmethacrylat, Methylacrylat-Bütylacrylat-Copolymere, Methylmethacrylat-Butylmethacrylat-Copolymere oder allgemein Polyalkylmethacrylate (PAMA), Ethylen-Vinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere, Celluloseacetat, PVB und PVC. Ein besonders bevorzugter Kunststoff ist dabei PVC.

Darüber hinaus können die erfindungsgemäßen Gemische zur Modifizierung von Kunststoffmischungen, beispielsweise der Mischung eines Polyolefins mit einem Polyamid, eingesetzt werden.

Zusammensetzungen aus Kunststoff/-en, insbesondere PVC oder PAMA, die erfindungsgemäße Gemische, die Ester der Formel **I** enthalten oder aus ihnen bestehen, können beispielsweise in folgenden Produkten enthalten sein: Gehäuse für Elektrogeräte, wie beispielsweise Küchengeräte, Computergehäuse, Gehäuse und Bauteile von Phono- und Fernsehgeräte, Rohrleitungen, Apparate, Kabel, Drahtummantelungen, Isolierbänder, im Innenausbau, im Fahrzeug- und Möbelbau, Plastisolen, in Bodenbelägen, medizinische Artikel, Lebensmittelverpackungen, Dichtungen, Folien, Verbundfolien, Schallplatten, Kunstleder, Spielzeug, Verpackungsbehälter, Klebebandfolien, Bekleidung, Beschichtungen, Beflockungen und Bedruckungen, Fasern für Gewebe, beschichtete Gewebe. Weiterhin können Zusammensetzungen aus Kunststoff, insbesondere PVC, die erfindungsgemäße Gemische, die Ester der Formel I enthalten oder aus ihnen bestehen, beispielsweise zur Herstellung folgender Erzeugnisse verwendet werden: Rohrleitungen, Schläuche, Kabel, Draht-Ummantelungen, Isolierbänder, im Fahrzeug- und Möbelbau, Plastisole, Profile, Bodenbeläge, medizinische Artikel (wie z. B. Blutbeutel, Schläuche, Infusionsbeutel etc), Spielzeuge, Lebensmittelverpackungen, Dichtungen, Folien, Verbundfolien, Platten, Kunstleder, Tapeten, Verpackungsbehälter, Klebebandfolien, Bekleidung, Beschichtungen oder Fasern für Gewebe, Schuhe, Unterbodenschutz, Nahtabdichtungen, Dachbahnen, Modelliermassen oder Bälle.

PVC-Zusammensetzungen oder Plastisole, die PVC und erfindungsgemäße Gemische, die Ester der Formel **I** enthalten oder aus ihnen bestehen, enthalten vorzugsweise von 5 bis 250 Massenteile, bevorzugt von 10 bis 200 Massenteile und besonders bevorzugt von 20 bis 100 Massenteile an den erfindungsgemäßen Gemischen pro 100 Massenteilen PVC.

**<u>Beispiel 1: Synthese von Isonansäure</u>**

**a) Herstellung von $C_9$-Aldehyden auf Basis von Dibuten**

Für die Herstellung von $C_9$-Aldehyden (Isononanalen) durch Hydroformylierung von $C_8$-Olefmen wurde als Edukt ein $C_8$-Olefin-Gemisch (Dibuten) aus dem Octol-Prozess der OXENO Olefinchemie GmbH eingesetzt. Das gewählte experimentelle Vorgehen wird im Folgenden dargestellt.

In einem 51 Hochdruckautoklaven mit Rührer und elektrischer Heizung wurden 2000 g Dibuten in Gegenwart eines Kobaltkatalysators bei 175 °C und einem Synthesegasdruck von 280 bar 3 Stunden lang hydroformyliert. Der Katalysator wurde hergestellt, indem 640 g einer wässrigen Kobaltacetat-Lösung mit 1,0 Massen-% Kobalt bei 170 °C und 28 MPa 6 Stunden mit Synthesegas behandelt wurden. Nach Abkühlen und Entspannen wurden die gebildeten

Kobaltcarbonyle durch Extraktion mit den 2000 g Di-n-buten in die organische Phase überführt, die von der wässrigen Phase abgetrennt wurde. Die Konzentration des Katalysators im Dibuten betrug 0,02 Massen-% bezogen auf das Dibuten und gerechnet als Kobaltmetall. Danach wurde bei 80 °C das Hydroformylierungsgemisch durch Behandlung mit 1000 g einer 5%igen wässrigen Essigsäure in Gegenwart von Luft, die über eine Fritte mit einem Durchsatz von 30 l/h über 30 Minuten dem Gemisch zugeleitet wurde, von Kobalt befreit. Das entkobaltete Hydroformylierungsgemisch wurde anschließend von der wässrigen Phase abgetrennt.

Das Verfahren wurde unter gleichen Reaktionsbedingungen viermal durchgeführt. Die entkobalteten Hydroformylierungsgemische wurden vereinigt. Es wurden 9450 g Gemisch erhalten. Der Rohproduktaustrag weist nach einer gaschromatographischen Analyse (GC-Analyse) folgende Zusammensetzung in Massen-% auf: 19,8 % $C_8$-Kohlenwasserstoffe, 57,6 % $C_9$-Aldehyde, 18,3 % $C_9$-Alkohole, 2,7 % $C_9$-Alkoholformiate und 1,6 % Rückstand.

Der Rohproduktaustrag wurde in einer nachgeschalteten Batch-Destillation von den nicht umgesetzten $C_8$-Kohlenwasserstoffen (Leichtsieder) befreit. Folgende typische Zusammensetzung der Sumpffraktion in Massen.-% wurde nach GC -Analyse ermittelt: 73,2 % $C_9$-Aldehyde, 21,5 % $C_9$-Alkohole, 3,1 % $C_9$-Alkohonbrmiate und 2,0 % Hochsieder und 0,2 % $C_8$-Kohlenwasserstoffe.

Die Sumpffraktion, die das Wertprodukt $C_9$-Aldehyde enthielt, wurde anschließend für die Herstellung der $C_9$-Carbonsäuren verwendet.

**b)** Herstellung von $C_9$-Säuren durch Oxidation von $C_9$-Aldehyden

Die Herstellung der $C_9$-Säuren durch Flüssigphasenoxidation der $C_9$-Aldehyde erfolgte in einem beheizbaren 61 Doppclmantel-Rührkcssel. Als Edukt wurde das Hydroformylierungsprodukt aus dem Beispiel 1a mit rd. 73 Massen-% $C_9$-Aldehyden verwendet.

Für einen Reaktionsansatz wurden im Reaktor 3500 g flüssiges Edukt vorgelegt. Als Reaktionsgas wurde ein Stickstoff-Sauerstoff-Gemisch verwendet, das in unterem Reaktorteil gleichmäßig über eine Fritte in die Flüssigkeit verteilt wurde.

In den Reaktor wurden ein konstanter Stickstoffstrom von 30 Nl/h und ein je nach dem Verbrauch durch die Reaktion über eine Online-Messung des Sauerstoffgehaltes im Abgas geregelter Sauerstoffstrom dosiert. In den Gasraum des Reaktors in oberem Reaktorteil wurde ein konstanter Stickstoffstrom von 330 Nl/h dosiert. Es wurde ein maximaler Sauerstoffgehalt im Abgas von 6 Vol.-% zugelassen. Die Oxidation der $C_9$-Aldehyde wurde bei einer Reaktionstemperatur von 55 °C und einem Druck von 0,12 MPa durchgeführt. Der Fortschritt der Oxidation wurde durch eine regelmäßige Probennahme und anschließende GC-Analyse ermittelt.

Unter den gewählten Reaktionsbedingungen wurde nach 20 Stunden Versuchszeit ein Roh-Produkt erhalten, dessen Zusammensetzung in Tabelle 1 in der zweiten Spalte aufgelistet ist.

Der Reaktionsaustrag der Oxidation wurde anschließend in einer Batch-Destillation aufgearbeitet. Für die Destillation wurde eine Laborpackungskolonne (Sulzer DX Packung) mit 5 l Blase verwendet. Die Zusammensetzung des Produktes vor und nach der Destillation ist in Tabelle 1 aufgeführt.

Tabelle 1: Produkt-Zusammensetzung

| Komponente | Roh-Produkt Oxidation Massen-% | Produkt nach Destillation In Massen-% |
|---|---|---|
| $C_8$-Kohlwasserstoffe | 0,96 | 0,03 |
| $C_9$-Aldchyde (Isononanale) | 5,20 | 0,13 |
| Ester (Isononylformiatc) | 2,96 | 0,02 |
| $C_9$-Alkohole (Isononanole) | 16,68 | 0,24 |
| $C_9$-Säuren ( Isononansäuren) | 71,33 | 99,36 |
| Hochsieder | 2,87 | 0,23 |

Wie der Tabelle 1 zu entnehmen ist, wurde für die im Folgenden beschriebene Umsetzung ein $C_9$-Carbonsäuregemisch hoher Reinheit gewonnen.

**Beispiel 2: Veresterung von Isononansäure aus Beispiele 1 mit Isosorbid**

**(erfindungsgemäß)**

In einem 2 Liter-Mehrhalskolben mit Rührer, Wasserabscheider, Tropftrichter, Innenthermometer und Tauchrohr wurden 365 g (2,5 Mol) Isosorbid (Fa. Cerestar) mit 1027 g (6,5 Mol) Isononansäure nach Beispiel 1 und 0,91 g Tctrabutyl-orthotitanat (0,25 Massen-% bezogen auf Isosorbid, Fa. DuPont, Tyzor TnBT) unter Rühren und Stickstoffeinperlung über das Tauchrohr (6 1/h) auf 230 °C erhitzt. Durch Anlegen eines leichten Vakuums wurde bei dieser Temperatur gewährleistet, dass das Reaktionswasser über den Wasserabscheider komplett ausgetragen werden konnte. Der Reaktionsverlauf wurde über GC-Analytik verfolgt. Nach insgesamt etwa 8 Stunden war die Reaktion beendet und der Wasserabscheider durch eine Destillationsbrücke ersetzt, über die bei einer Temperatur von 200 bis 230 °C die überschüssige Isononansäure abdestilliert werden konnte. Auch hierbei wurde sukzessive der Druck bis etwa 3 hPa reduziert. Danach wurde der Ansatz auf 80 °C abgekühlt und mit 50 ml 10%iger Natronlauge neutralisiert. Danach wurde der Rohester in einem Scheidetrichter drei Mal mit je 300 ml 5%iger NaCl-Lösung gewaschen und die wässrige Phase jeweils abgetrennt und verworfen. Danach wurde der Ansatz mit 1 Massen% Aktivkohle versetzt und im Rührkolben bei 125 °C und einem Druck von 5 hPa eine Stunde getrocknet und nach Abkühlen auf 100 °C filtriert. Wegen der intensiven Färbung des Produktes wurde dann der Ansatz im Rührkolben nochmals bei 90 °C mit 2 Massen-% $H_2O_2$ (35%ig, Fa. Merck) gebleicht, anschließend nochmals neutralisiert (40 ml 10%ige NaOH), danach wieder zwei Mal gewaschen, getrocknet und filtriert. Da die Farbe noch immer unbefriedigend war, wurde die Sequenz aus $H_2O_2$-Bleichung, Neutralisation, waschen, trocknen und filtrieren wie oben beschrieben noch zwei Mal wiederholt. Das so erhaltene Produkt Isosorbid-diisononanoat (IsDIN) hatte eine Farbzahl nach Hazen von APHA 62. Die Reinheit, bestimmt über GC-Analytik, lag bei 99 % (Ester A). Der über NMR bestimmte Verzweigungsgrad der Seitenkette lag bei 1,3.

**Beispiel 3: Veresterung von 2-Ethylhexansäure mit Isosorbid (Vergleichsbeispiel)**

In einem 2 Liter-Mehrhalskolben mit Rührer, Wasserabscheider, Tropftrichter, Innenthermometer und Tauchrohr wurden 292 g (2 Mol) Isosorbid (Fa. Cerestar) mit 730 g (5 Mol) 2-Ethylhexansäure (Fa. European Oxo) und 1,46 g Tetrabutyl-orthotitanat (0,5 Massen-% bezogen auf Isosorbid, Fa. DuPont, Tyzor TnBT) unter Rühren und Stickstoffeinperlung über das Tauchrohr (6 l/h) auf 240 °C erhitzt. Durch Anlegen eines leichten Vakuums wurde bei dieser Temperatur gewährleistet, dass das Reaktionswasser über den Wasserabscheider komplett ausgetragen werden konnte. Der Reaktionsverlauf wurde über GC-Analytik verfolgt. Nach insgesamt etwa 8 Stunden war die Reaktion beendet und der Wasserabscheider wurde darin durch eine Destillationsbrücke ersetzt, über die bei einer Temperatur von 180 °C die überschüssige 2-Ethylhexansäure bei einem Druck bis minimal 5 hPa abdestilliert werden konnte. Anschließend wurde der Rückstand mit 2,5 Massen-% $H_2O_2$-Lösung (35%ig, Merck) bei 80 bis 90 °C gebleicht, anschließend entsprechend Beispiel 2 mit NaOH-Lösung neutralisiert, dann gewaschen und getrocknet. Zur weiteren Farbverbesserung wurde dann nochmals mit der gleichen Menge $H_2O_2$ gerührt, dann bei 120 °C getrocknet, danach noch zwei Mal mit 5 %iger NaCl-Lösung gewaschen, danach nochmals bei 120 °C getrocknet, mit 2 % Aktivkohle eine weitere Stunde bei dieser Temperatur gerührt und anschließend filtriert. Der so erhaltene Isosorbid-di-2-Ethylhexanoylester (IsDEH, Ester B) hatte eine Reinheit von ca. 99 % (GC) und eine Hazen-Farbzahl von 50, letztere gemessen in Anlehnung an DIN EN ISO 6271 mit einem Farbzahlmessgerät LICO 400 der Fa. Hach-Lange.

**Beispiel 4: Veresterung von 3,5,5-Trimethylhexansäure mit Isosorbid**

**(Versteichsbeispiel)**

In analoger Vorgehensweise zur Synthese ausgehend von der Isononansäure aus Beispiel 1 wurde auch der Di-3,5,5-Trimethylhexylester des Isosorbids hergestellt. Als Ausgangsprodukt wurde kommerziell erhältliche 3,5,5-Trimethylhexansäure ("Isononansäure", Fa. European Oxo) eingesetzt.

Der so hergestellte Ester Isosorbid-di-3,5,5-trimethylhexanoat (IsD355TMH) konnte nach Durchführung diverser Reinigungsschritte (s. Beispiele 2 und 3) in einer Reinheit von 98,2 % mit einer Hazen-Farbzahl von 68 (Methode s. Beispiel 3) hergestellt werden. Der Schmelzpunkt dieses Esters, gemessen mit DSC (Onset), lag bei 21,7 °C. Im Folgenden wird er als Ester C bezeichnet

**Beispiel 5:Veresterung von Pelargonsäure (n-Nonansäure) mit Isosorbid**

**(Vergleichsbeispiel)**

In einem 4 Liter-Mehrhalskolben mit Rührer, Wasserabscheider, Tropftrichter, Innenthermometer und Tauchrohr wurden 876 g (6 Mol) Isosorbid (Fa. Cerestar) mit 2370 g (15 Mol) Pelargonsäure (Fa. FLUKA) und 2,19 g Tetrabutylorthotitanat (0,25 Massen-% bezogen auf Isosorbid, Fa. DuPont, Tyzor TnBT) unter Rühren und Stickstoffeinperlung über das Tauchrohr (6 1/h) auf 220 °C erhitzt. Durch Anlegen eines leichten Vakuums wurde bei dieser Temperatur gewährleistet, dass das Reaktionswasser über den Wasserabscheider komplett ausgetragen werden konnte. Der Reaktionsverlauf wurde über GC-Analytik verfolgt. Nach insgesamt etwa 8,5 Stunden war die Reaktion beendet und der

Wasserabscheider wurde durch eine Destillationsbrücke ersetzt, über die bei einer Temperatur von 180 °C und einem sukzessive bis auf 2 hPa erniedrigten Druck der überwiegende Teil der überschüssigen Pelargonsäure abdestilliert werden konnte. Daran schlossen sich eine Wasserdampfdestillation bei 180 °C und eine Trocknung in Gegenwart von Aktivkohle (1 Massen-%) an. Anschließend wurde analog zu Beispiel 2 neutralisiert, der Ansatz zwei Mal mit 250 ml 5%iger Nacl-Lösung gewaschen und nach einer erneuten Wasserdampfdestillation im Vakuum getrocknet und anschließend filtriert. Zur weiteren Aufhellung wurde dann bei einer Temperatur von 80 bis 120 °C mit 2 % H2O2 gerührt, anschließend nochmals neutralisiert, noch zwei Mal gewaschen und nach einer letzten Wasserdampfdestillation bei 140 °C im Vakuum nach Zugabe von 1 % Aktivkohle getrocknet und der Ansatz dann filtriert. Der resultierende Ester Isosorbid-dipelargonat (IsDnN) hatte dann eine Hazen-Farbzahl von APHA 38 (Methode s. Beispiel 3) und einen mittels GC bestimmten Gehalt von 99 %.

Beim Abkühlen auf Raumtemperatur wurde der Ester fest, eine Bestimmung des Schmelzpunktes mittels DSC (Differential Scanning Colorimetry) ergab einen Wert von 27 °C. Die Tatsache, dass dieser Ester (Ester D) bei Raumtemperatur ein Feststoff ist, schließt seine Verwendung als alleinigen Weichmacher für Plastisole praktisch aus, da die zur Verarbeitung (Streichen, Tauchen, Sprühen, Rotieren) notwendige pastenartige Konsistenz nur schwer durch Zugabe von weiteren Hilfsmitteln machbar wäre. Daher wurde dieser Ester bei den weiteren Untersuchungen nicht mehr eingesetzt.

Beispiel 6: Herstellung von Isosorbidestern auf Basis von Gemischen von Pelargonsäure und 3,5,5-Trimethylhexansäure

Die Tatsache, dass die in den Beispielen 4 und 5 hergestellten Ester Schmelzpunkte im Bereich der Umgebungstemperatur aufweisen, lässt sie für die Plastisolverarbeitung als ungeeignet erscheinen. Eine Reduzierung der Kristallisationsneigung sollte durch Mischen der beiden Säuren und anschließende Veresterung erreicht werden können. Zu diesem Zwecke wurden gemäß Tabelle 2 Mischungen von Pelargon- und 3,5,5-Trimethylhexansäure hergestellt und diese nach den bereits oben beschriebenen Verfahren verestert. In der Tabelle sind neben den molaren Anteilen der Säuren im Reaktionsansatz auch die mittels GC erhaltenen Gehalte an Isosorbiddiestern und deren Schmelzpunkte, zusammen mit den entsprechenden Werten der Ester aus den Beispielen 4 und 5 aufgeführt.

Die Schmelzpunkte wurden mittels DSC bestimmt, hierzu wurde jeweils der Anstieg des Schmelzsignals (sog. "Onset") herangezogen. Im Falle von mehreren Schmelzpunkten (verschiedene Phasen, z. B. bei Ester F) wurde der höchste Schmelzpunkt aufgeführt, da unterhalb dieser Temperatur die ersten Kristallisationen einsetzen. Alle Ester weisen darüber hinaus auch einen Glaspunkt auf, der auf mehr oder weniger hohe amorphe Anteile hinweist.

Tabelle 2:

| Beispiel Nr. | Anteil Pelargonsäure im Ansatz in Mol-% | Anteil 3,5,5-Trimethylhexansäure im Ansatz in Mol-% | Gehalt in % | Schmelzpunkt der Isosorbidester |
|---|---|---|---|---|
| D | 100 | 0 | 99 | 27 °C |
| E | 95 | 5 | 98,8 | 26,3 |
| F | 60 | 40 | 98,4 | 4 °C |
| G | 5 | 95 | 98 | 22,4 °C |
| C | 0 | 100 | 98,2 | 21,7 °C |

Die Ester, bei denen eine der Säuren mit einem Anteil von 95 oder mehr Mol-% zugegen ist, können auf Grund ihrer hohen Schmelzpunkte bei Raumtemperatur nicht oder nur mit unverhältnismäßig hohem Aufwand in Plastisolverfahren eingesetzt werden. Der erfindungsgemäße Ester F wiederum kann ohne Probleme bei Raumtemperatur verarbeitet werden.

**Beispiel 7: Hersteliung von Plastisolen**

Die verwendeten Einwaagen der Komponenten für die verschiedenen Plastisole sind der nachfolgenden Tabelle zu entnehmen.

Tabelle 3: Rezepturen gemäß Beispiel (Alle Angaben in phr (= Massenteile pro 100 Massenteile PVC))

| **Plastisolrezeptur** | **1** | **2** | **3** |
|---|---|---|---|
| Vestolit B 7021 (Vestolit GmbH) | 100 | 100 | 100 |
| Vestinol 9 (DINP der OXENO Olefinchemie ) | 50 | | |
| Isosorbid-diisononanoytester (IsDIN aus Beispiel 2, erfindungsgemäß) | | 50 | |
| Isosorbid-di-2-ethylhexanoylester (IsDEH aus Beispiel 3, Vergleichsbeispiel) | | | 50 |
| Epoxidiertes Sojabohnenöl (Drapex 39, Fa. Crompton) | 3 | 3 | 3 |
| Mark CZ 140 (Fa. Crompton) | 1,5 | 1,5 | 1,5 |

Die Weichmacher wurden vor der Zugabe auf 25 °C temperiert. Zuerst wurden die flüssigen Bestandteile und dann die pulverförmigen in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

**Beispiel 8: Messung der Gelierkurven**

Die Untersuchung des Gellerverhaltens der Plastisole wurde in einem Oszillationsviskosimctcr der Marke Bohlin CVO (Meßsystem PP20), welches schubspannungsgesteuert betrieben wurde, vorgenommen.

Folgende Parameter wurden eingestellt:

Modus: Temperatur-Gradient
Start-Temperatur: 25 °C
End-Temperatur: 180 °C
Heiz/Kühlrate: 2 °C/min
Temperatur nach der Messung: 25 °C
Oszillations-Frequenz: 2 Hz
Verzögerungszeit: 1 s
Wartezeit: 15 s
Kontinuierliche Oszillation: an
Automatische Schubspannungsvorgabe: an
Startschubspannung: 0,3 Pa
Soll-Deformation: 0,002
Spaltweite 0,5 mm

**Durchführung der Messung:**

Auf die untere Meßsystemplatte wurde mit dem Spatel ein Tropfen der zu messenden Plastisolrezeptur luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Schutzabdeckung, die auch der Wärmeisolierung dient, aufgelegt und die Messung gestartet.

In Figur 1 wurde die sog. komplexe Viskosität der Plastisole in Abhängigkeit von der Temperatur aufgetragen. Das erfindungsgemäße Plastisol 2 (IsDIN) ist durch die durchgezogene Linie mit ausgefüllten Kreisen, das Plastisol 1 (Vestinol 9) durch die gestrichelte Linie mit Rauten und das Vergleichsbeispiel mit IsDEH als Weichmacher durch die gepunktete Linie mit Dreiecken dargestellt. Ein Einsetzen des Geliervorganges ist generell in einem plötzlichen starken Anstieg der komplexen Viskosität zu erkennen. Je früher dieser Viskositätsanstieg einsetzt, desto besser ist generell die Gelierfähigkeit des Systems.

Ergebnis: Die Gelierung des Plastisols mit dem erfindungsgemäßen Ester IsDIN ist vergleichbar mit der auf Basis des korrespondierenden Phthalats DINP und auch nur wenig langsamer als im Falle des IsDEH.

**Beispiel 9: Messung der Plastisol-Viskositäten**

Die Messung der Viskositäten der in Beispiel 7 hergestellten Plastisole wurden in Anlehnung an die DIN 53 019 mit einem Rheometer Physica DSR 4000 (Fa. Paar-Physica), welches über die zugehörige Software US 200 gesteuert wird, wie folgt durchgeführt.

Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die o. g. Software. Folgende Punkte wurden angesteuert:

- Eine Vorscherung von 100 $s^{-1}$ für den Zeitraum von 60 s, bei der keine Messwerte aufgenommen wurden
- Eine Abwärtsrampe, beginnend bei 200 $s^{-1}$ bis herunter zu 0,1 $s^{-1}$, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Die Messungen wurden jeweils nach 2 h, 4 h, 24 h und 28 Tagen durchgeführt. Zwischen diesen Zeitpunkten wurde die Paste bei 25 °C gelagert.

In der nachfolgenden Tabelle 4 sind exemplarisch für die Schergeschwindigkeit von 100 $s^{-1}$ jeweils die nach den angegebenen Lagerzeiten erhaltenen entsprechenden Viskositätswerte aufgeführt.

Tabelle 4: Schergeschwindigkeit 100 $s^{-1}$ (Angaben der Viskositäten in Pa*s)

| Plastisol-rezeptur | Verwendeter Weichmacher | 2 h | 24 h | 7 d | 28 d | Gesamtanstieg von 2 h nach 28 Tagen in % |
|---|---|---|---|---|---|---|
| 1 | DINP (Vestinol 9) | 3,44 | 3,88 | 4,1 | 5,12 | 49 % |
| 2 | IsDIN aus Beispiel 2 | 6,34 | 6,63 | 7,32 | 7,58 | 20 % |
| 3 | IsDEH aus Beispiel 3 | 6,42 | 6,62 | 7,4 | 8,09 | 26 % |

Die Viskosität des erfindungsgemäßen Plastisols 2 ist vergleichbar mit der des Vergleichsbeispiels mit IsDEH als Weichmacher. Das erfindungsgemäße Plastisol zeigt die beste Lagerstabilität.

**Beispiel 10: Herstellung von Folien**

Zur Erzeugung der Prütkörper wurden zunächst für jede Rezeptur aus Tabelle 3 l mm dicke Folien hergestellt. Hierzu wurde zunächst Hochglanztrennpapier (Fa. Sappi, Italien) auf eine Größe von 30* 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssige Paste aufzufangen. Danach wurde die Paste vor der Rakel aufgetragen und diese durch Ziehen des Führungsrahmens mit der Rakel über das eingespannte Trennpapier verstrichen (ca. 6 m/min Geschwindigkeit). Danach wurde die Rakel herausgenommen und der Klebestreifen mit der überschüssigen Paste abgenommen. Anschließend wurde die Schmelzwalze abgesenkt und der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

**Beispiel 11: Messung der Flüchtigkeit aus der Folie in Anlehnung an DIN 53 407**

Aus den in Beispiel 10 hergestellten Folien mit einer Dicke von etwa 1 mm wurden jeweils drei Rundscheiben mit einem Durchmesser von 50 mm ausgestanzt und zunächst 24 h im Normklima (23 °C / 50 % relative Luftfeuchtigkeit) gelagert und dann verwogen. Danach wurden die Rundscheiben in Anlehnung an die DIN 53 407 (Verfahren A, direkter Kontakt mit Aktivkohle, Körnung 2,5 mm) jeweils 24 Stunden im Heizschrank bei 80 °C erwärmt.

Danach wurden die Scheiben wieder dem Heizschrank entnommen, im Normklima bei 24 Stunden abgekühlt und wieder verwogen, bevor sie erneut im Heizschrank gelagert wurden. Nach einer Lagerperiode von 7 * 24 Stunden wurde der Test beendet. In Tabelle 5 sind die erhaltenen Messwerte aufgeführt:

Tabelle 5: Ergebnisse der Flüchtigkeitsmessung

| Plastisol-rezeptur | Verwendeter Weichmacher | 1 d | 2 d | 3 d | 4 d | 5 d | 6 d | 7 d |
|---|---|---|---|---|---|---|---|---|
| 1 | Vestinol 9 (DINP) | 0.7 | 1,1 | 1,53 | 1,86 | 2,19 | 2,4 | 2,74 |
| 2 | IsDIN aus Beispiel 2 | 0,86 | 1,24 | 1,57 | 1,91 | 2,27 | 2,47 | 2,79 |
| 3 | IsDEH aus Beispiel 3 | 1,34 | 2,47 | 3,8 | 4,85 | 5,9 | 6,69 | 7,74 |

Ergebnis: Die Flüchtigkeit der aus den erfindungsgemäßen Estern hergestellten Folien entspricht denen, die aus dem korrespondierenden Phthalat DINP hergestellt worden sind und ist deutlich niedriger als die des Vergleichsproduktes IsDEH und von IsD355TMH.

**Beispiel 12: Messung der Gtasubersansstemperaturen der Folien**

Aus den nach Beispiel 10 hergestellten Folien wurden 60 mm lange, 80 mm breite und 1 mm dicke Stücke ausgestanzt und von diesen in einem Torsionspendel vom Typ MYRENNE ATM III nach DIN EN ISO 6721 (Teil 2) bei Temperaturen von -100 °C bis +100 °C und einer Frequenz von 1 $s^{-1}$ jeweils die Steifigkeit G' und der Verlustmodul G" bestimmt.

Aus dem Maximum von G" ließ sich die Glasübergangstemperatur $T_G$ bestimmen. Diese ist ein Maß für die Flexibilität bei tiefen Temperaturen.

Die Glasübergangstemperaturen der Prüfkörper sind in Tabelle 6 aufgelistet:

Tabelle 6: Glasübergangstemperaturen der Folien

| Folien aus Plastisolrezeptur Nr. | Verwendeter Weichmacher | Glasübergangstemperatur $T_G$ |
|---|---|---|
| 1 | Vestinol 9 (DINP) | - 31°C |
| 2 | IsDIN aus Beispiel 2 | - 16°C |
| 3 | IsDEH aus Beispiel 3 | - 11°C |

Die Glasübergangstemperatur der Folien hergestellt aus den erfindungsgemäßen Diisononanoylestem ist deutlich niedriger als die der Folie hergestellt aus IsDEH (Vergleichsbeispiel), wenn auch schlechter als die des korrespondierenden Phthalates DINP.

Fazit:

Es kann somit festgestellt werden, dass die erfindungsgemäßen Isosorbid-diisononanoylester (IsDIN) gute Weichmachereigenschaften zeigen und den Isosorbidestern hergestellt aus 2-Ethylhexansäure (IsDEH, Beispiel 3), 3,5,5-Trimethylhexansäure (IsD355TMH, Beispiel 4) und Pelargonsäure (IsDnN, Beispiel 5) anwendungstechnisch überlegen sind.

**Patentansprüche**

1. Gemisch, enthaltend Diester eines Dianhydrohexitolderivates mit Carbonsäuren der Summenformel $C_8H_{17}COOH$ der Formel **I**

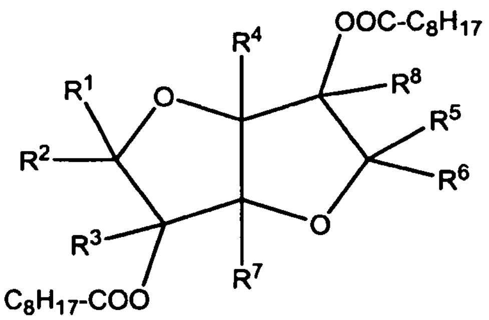

I

mit $R^1$ bis $R^8$ = H oder Alkylgruppe mit 1 bis 6 Kohlenstoffatomen, wobei die Reste $R^1$ bis $R^8$ gleich oder unterschiedlich sein können,
**dadurch gekennzeichnet,**
**dass** im Gemisch zumindest zwei unterschiedliche Diester **I** enthalten sind, die sich in der Konstitution zumindest eines der vorhandenen Carbonsäurereste $C_8H_{17}COO$ unterscheiden.

2. Gemisch gemäß Anspruch 1,
**dadurch gekennzeichnet,**
**dass** das durch Verseifung der im Gemisch enthaltenen Diester erhaltene Carbonsäuregemisch mindestens zwei Carbonsäuren der Summenformel $C_8H_{17}COOH$ mit unterschiedlicher Konstitutionsformel aufweist, wobei keine der im Gemisch vorhandenen Carbonsäuren mit einem Anteil von mehr als 95 Mol% in dem Carbonsäuregemisch vorhanden ist.

3. Gemisch gemäß Anspruch 1 oder 2,
**dadurch gekennzeichnet,**
**dass** die durch Verseifung der im Gemisch enthaltenen Diester erhaltenen Carbonsäuren der Summenformel $C_8H_{17}COOH$ weniger als 10 Mol % 3,5,5-Trimethylhexansäure enthalten.

4. Gemisch nach zumindest einem der Ansprüche 1 bis 3,
**dadurch gekennzeichnet,**
**dass** die Carbonsäurereste $C_8H_{17}COO$ der im Gemisch enthaltenen Diester einen Verzweigungsgrad von 0,7 bis 2,0 aufweisen.

5. Gemisch nach zumindest einem der Ansprüche 1 bis 4,
**dadurch gekennzeichnet,**
**dass** die Reste $R^1$, bis $R^8$ jeweils H sind.

6. Gemisch nach zumindest einem der Ansprüche 1 bis 5,
**dadurch gekennzeichnet,**
**dass** die Diester des Gemisches mindestens zwei verschiedene bicyclische Unterstrukturen von Formel I aufweisen, die sich durch ihre Konfiguration unterscheiden.

7. Gemisch nach zumindest einem der Ansprüche 1 bis 6,
**dadurch gekennzeichnet,**
**dass** die Diester der Formel **I** im Wesentlichen Diester der Formel **Ia**

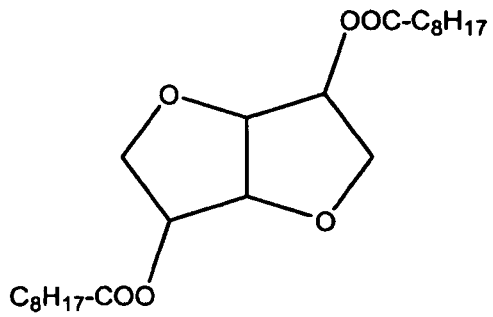

Ia

sind, wobei die chiralen C-Atome des bicyclischen Grundgerüstes unabhängig voneinander R oder S konfiguriert sein können.

**8.** Gemisch nach Anspruch 7,
**dadurch gekennzeichnet,**
**dass** die Diester im Wesentlichen Diester des Isosorbids sind.

**9.** Gemisch nach zumindest einem der Ansprüche 1 bis 8,
**dadurch gekennzeichnet,**
**dass** es zumindest ein Polymer und/oder mindestens einen weiteren Weichmacher, der kein Diester der Formel I ist, aufweist.

**10.** Gemisch nach Anspruch 9,
**dadurch gekennzeichnet,**
**dass** das Mol-Verhältnis von weiterem Weichmacher zu den Diestern der Formel **I** von 1 zu 10 bis 10 zu 1 beträgt.

**11.** Verfahren zur Herstellung von Diestern der Formel **I**

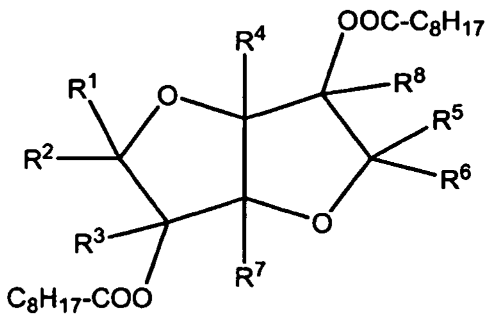

I

mit $R^1$ bis $R^8$= H oder Alkylgruppe mit 1 bis 6 Kohlenstoffatomen, wobei die Reste $R^1$ bis $R^8$ gleich oder unterschiedlich sein können,
**dadurch gekennzeichnet,**
**dass** ein sechswertiger Alkohol der Formel **II**,

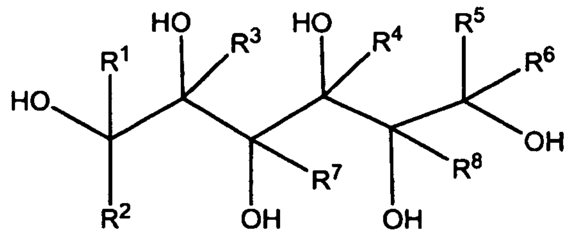

II

wobei die Reste $R^1$ bis $R^8$ die in Formel I genannte Bedeutung haben, und/oder ein Anhydro- oder Dianhydro-Derivat eines Alkohols der Formel **II** mit einem Gemisch, das zumindest zwei unterschiedliche Carbonsäuren der Summenformel $C_8H_{17}COOH$ aufweist, umgesetzt wird.

**12.** Verfahren nach Anspruch 11,
**dadurch gekennzeichnet,**
**dass** ein Gemisch von isomeren Carbonsäuren der Summenformel $C_8H_{17}COOH$ eingesetzt wird, das einen mittleren Verzweigungsgrad von 0,7 bis 2,0 aufweist.

**13.** Verfahren nach zumindest einem der Ansprüche 11 bis 12,
**dadurch gekennzeichnet,**
**dass** die Veresterung in Gegenwart eines Katalysators durchgeführt wird.

**14.** Verwendung der Gemische gemäß einem der Ansprüche 1 bis 10 in Farben, Tinten oder Lacken, in Plastisolen, Klebstoffen oder Klebstoffkomponenten, in Dichtungsmassen, als Weichmacher in Kunststoffen oder Kunststoffkomponenten, als Lösemittel, als Schmierölkomponente und als Hilfsmittel bei der Metallverarbeitung.

**15.** PVC-Zusammensetzung, enthaltend PVC und von 5 bis 250 Massenteilen eines Gemisches gemäß einem der Ansprüche 1 bis 10 pro 100 Massenteile PVC.

**16.** Plastisol, enthaltend PVC und von 5 bis 250 Massenteilen eines Gemisches gemäß einem der Ansprüche 1 bis 6 pro 100 Massenteile PVC.

## Claims

**1.** Mixture, comprising diesters of a dianhydrohexitol derivative with carboxylic acids of the empirical formula $C_8H_{17}COOH$ of the formula **I**

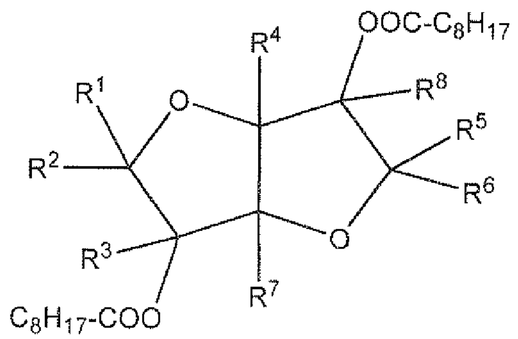

I

where $R^1$ to $R^8$ = H or alkyl group having from 1 to 6 carbon atoms, $R^1$ to $R^8$ being identical or different,
**characterized in that**
in the mixture at least two different diesters **I** are present which differ in the constitution of at least one of the carboxylic acid radicals $C_8H_{17}COO$ present.

2. Mixture according to Claim 1,
**characterized in that**
the carboxylic acid mixture obtained via saponification of the diesters present in the mixture comprises at least two carboxylic acids of the empirical formula $C_8H_{17}COOH$ with a different constitutional formula, the proportion present of any of the carboxylic acids present in the mixture not being more than 95 mol% in the carboxylic acid mixture.

3. Mixture according to Claim 1 or 2,
**characterized in that**
the carboxylic acids of the empirical formula $C_8H_{17}COOH$ obtained via saponification of the diesters present in the mixture comprise less than 10 mol% of 3,5,5-trimethylhexanoic acid.

4. Mixture according to at least one of Claims 1 to 3,
**characterized in that**
the carboxylic acid radicals $C_8H_{17}COO$ of the diesters present in the mixture have a degree of branching of from 0.7 to 2.0.

5. Mixture according to at least one of Claims 1 to 4,
**characterized in that**
each of the radicals $R^1$ to $R^8$ is H.

6. Mixture according to at least one of Claims 1 to 5,
**characterized in that**
the diesters of the mixture comprise at least two different bicyclic substructures of formula **I**,
which differ via their configuration.

7. Mixture according to at least one of Claims 1 to 6,
**characterized in that**
the diesters of the formula **I** are in essence diesters of the formula **Ia**

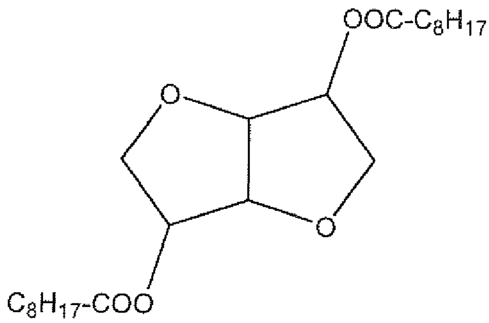

**Ia**

where the chiral C atoms of the underlying bicyclic skeleton can, independently of each other, have R or S configuration.

8. Mixture according to Claim 7,
**characterized in that**
the diesters are in essence diesters of isosorbide.

9. Mixture according to at least one of Claims 1 to 8,
**characterized in that**
it comprises at least one polymer and/or at least one further plasticizer which is not a diester of the formula **I**.

10. Mixture according to Claim 9,
**characterized in that**
the molar ratio of further plasticizer to the diesters of the formula **I** is from 1:10 to 10:1.

**11.** Process for preparation of diesters of the formula **I**

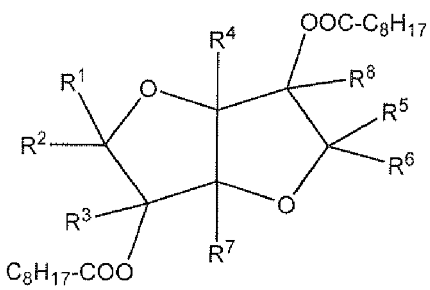

I

where $R^1$ to $R^8$ = H or alkyl group having from 1 to 6 carbon atoms, $R^1$ to $R^8$ being identical or different,
**characterized in that**
a hexahydric alcohol of the formula **II**

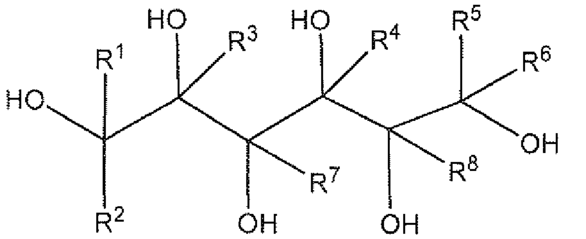

II

where the radicals $R^1$ to $R^8$ are as defined in formula **I**, and/or an anhydro or dianhydro derivative of an alcohol of the formula **II** is reacted with a mixture which comprises at least two different carboxylic acids of the empirical formula $C_8H_{17}COOH$.

**12.** Process according to Claim 11,
**characterized in that**
a mixture of isomeric carboxylic acids of the empirical formula $C_8H_{17}COOH$ is used whose average degree of branching is from 0.7 to 2.0.

**13.** Process according to at least one of Claims 11 to 12,
**characterized in that**
the esterification reaction is carried out in the presence of a catalyst.

**14.** Use of the mixtures according to any one of Claims 1 to 10 in paints, inks or coatings, in plastisols, adhesives or components of adhesives, in sealing compositions, as plasticizers in plastics or components of plastics, as solvents, as component of lubricating oils and as auxiliaries during metalworking.

**15.** PVC composition, comprising PVC and from 5 to 250 parts by weight of a mixture according to any one of Claims 1 to 10 per 100 parts by weight of PVC.

**16.** Plastisol, comprising PVC and from 5 to 250 parts by weight of a mixture according to any one of Claims 1 to 6 per 100 parts by weight of PVC.

## Revendications

**1.** Mélange, contenant des diesters d’un dérivé de dianhydrohexitol avec des acides carboxyliques de formule brute $C_8H_{17}COOH$ de formule I

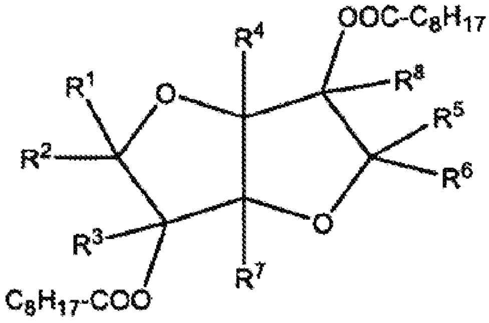

I

avec $R^1$ à $R^8$ = H ou un groupe alkyle comprenant 1 à 6 atomes de carbone, où les radicaux $R^1$ à $R^8$ peuvent être identiques ou différents, **caractérisé en ce que** le mélange contient au moins deux diesters I différents, qui se distinguent dans la constitution d'au moins un des radicaux acide carboxylique $C_8H_{17}COO$ présents.

2. Mélange selon la revendication 1, **caractérisé en ce que** le mélange d'acides carboxyliques obtenu par saponification des diesters contenus dans le mélange présente au moins deux acides carboxyliques de formule brute $C_8H_{17}COOH$ présentant une formule de constitution différente, où aucun des acides carboxyliques présents dans le mélange n'est présent en une proportion en plus de 95% en mole dans le mélange d'acides carboxyliques.

3. Mélange selon la revendication 1 ou 2, **caractérisé en ce que** les acides carboxyliques de formule brute $C_8H_{17}COOH$ obtenus par saponification des diesters contenus dans le mélange contiennent moins de 10% en mole d'acide 3,5,5-triméthylhexanoïque.

4. Mélange selon au moins l'une quelconque des revendications 1 à 3, **caractérisé en ce que** les radicaux d'acide carboxylique $C_8H_{17}COO$ des diesters contenus dans le mélange présentent un degré de ramification de 0,7 à 2,0.

5. Mélange selon au moins l'une quelconque des revendications 1 à 4, **caractérisé en ce que** les radicaux $R^1$ à $R^8$ représentent à chaque fois H.

6. Mélange selon au moins l'une quelconque des revendications 1 à 5, **caractérisé en ce que** les diesters du mélange présentent au moins deux sous-structures bicycliques différentes de formule I, qui se distinguent par leur configuration.

7. Mélange selon au moins l'une quelconque des revendications 1 à 6, **caractérisé en ce que** les diesters de formule I sont essentiellement des diesters de formule la

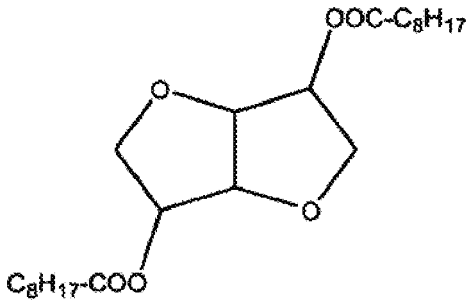

Ia

où les atomes de C chiraux de la structure de base bicyclique peuvent être configurés indépendamment l'un de l'autre en R ou en S.

**8.** Mélange selon la revendication 7, **caractérisé en ce que** les diesters sont essentiellement des diesters de l'isosorbide.

**9.** Mélange selon au moins l'une quelconque des revendications 1 à 8, **caractérisé en ce qu'**il présente au moins un polymère et/ou au moins un autre plastifiant, qui n'est pas un diester de formule I.

**10.** Mélange selon la revendication 9, **caractérisé en ce que** le rapport molaire de l'autre plastifiant aux diesters de formule I est de 1:10 à 10:1.

**11.** Procédé pour la préparation de diesters de formule I

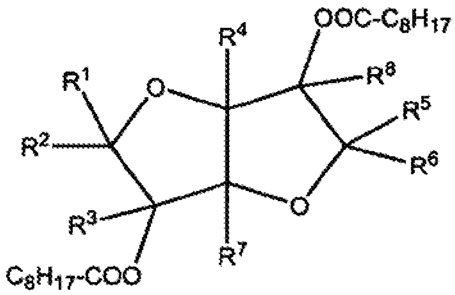

I

avec $R^1$ à $R^8$ = H ou un groupe alkyle comprenant 1 à 6 atomes de carbone, où les radicaux $R^1$ à $R^8$ peuvent être identiques ou différents, **caractérisé en ce qu'**on transforme un alcool hexavalent de formule II,

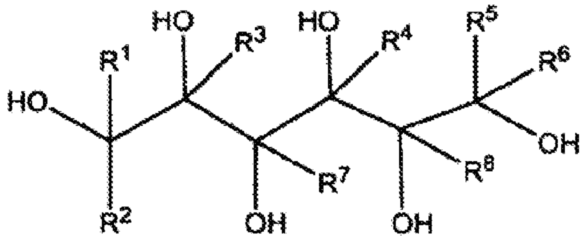

II

où les radicaux $R^1$ à $R^8$ présentent la signification mentionnée dans la formule I, et/ou un dérivé anhydro ou dianhydro d'un alcool de formule II avec un mélange qui présente au moins deux acides carboxyliques différents de formule brute $C_8H_{17}COOH$.

**12.** Procédé selon la revendication 11, **caractérisé en ce qu'**on utilise un mélange d'acides carboxyliques isomères de formule brute $C_8H_{17}COOH$, qui présente un degré de ramification moyen de 0,7 à 2,0.

**13.** Procédé selon au moins l'une quelconque des revendications 11 à 12, **caractérisé en ce que** l'estérification est réalisée en présence d'un catalyseur.

**14.** Utilisation des mélanges selon l'une quelconque des revendications 1 à 10 dans des encres, des teintures ou des laques, dans des plastisols, des adhésifs ou des composants d'adhésifs, dans des masses de bouchage, comme plastifiants dans des matériaux synthétiques ou des composants de matériaux synthétiques, comme solvants, comme composants de lubrifiants et comme adjuvants lors de la transformation de métaux.

**15.** Composition de PVC, contenant du PVC et 5 à 250 parties en masse d'un mélange selon l'une quelconque des revendications 1 à 10 par 100 parties en masse de PVC.

**16.** Plastisol, contenant du PVC et 5 à 250 parties en masse d'un mélange selon l'une quelconque des revendications 1 à 6 par 100 parties en masse de PVC.

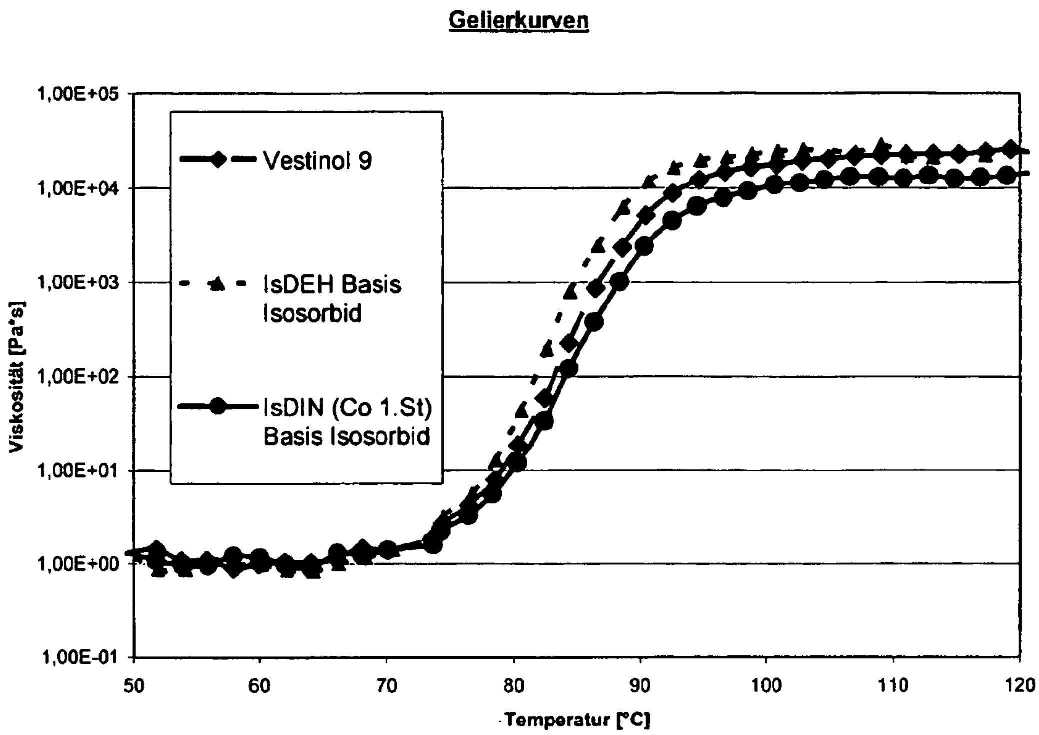
Gelierkurven
1,00E+05
1,00E+04
1,00E+03
1,00E+02
1,00E+01
1,00E+00
1,00E-01
Viskosität [Pa*s]
Vestinol 9
IsDEH Basis Isosorbid
IsDIN (Co 1.St) Basis Isosorbid
50
60
70
80
90
100
110
120
Temperatur [°C]

Figur 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9945060 A
- WO 0183488 A
- WO 03029339 A
- US 6284938 B
- US 6080903 A
- US 6072093 A
- US 6025533 A
- US 5990367 A
- US 5895830 A
- US 5856604 A
- US 5847252 A
- US 5081086 A
- EP 0395857 A
- EP 1029839 A
- EP 1171413 A
- EP 0850905 A
- EP 1172349 A
- EP 0213639 A
- EP 1201675 A
- WO 0316320 A
- WO 0316321 A
- WO 2005090276 A
- WO 2004020380 A
- DE 10327435
- WO 2006103338 A
- EP 1278752 A
- WO 06103338 A
- EP 1186593 A
- EP 1300388 A


### In der Beschreibung aufgeführte Nicht-Patentliteratur


- **Hachihama, Y. et al.** Preparation of plasticizers from carbohydrate sources. I. Levulinic acid esters. II. Sorbide esters. *TECHNOLOGY REPORTS OF THE OSAKA UNIVERSITY,* 1953, vol. 3 (72), 191-200
- **Haveren et al.** *ACS symposium series,* 2006, vol. 921, 99-115
- B. Ullmann's Enzyklopädie der Technischen Chemie. 1986, vol. 10, 254-260